# EUROPEAN PATENT SPECIFICATION

(11) **EP 2 937 102 B1**
(45) Date of publication and mention of the grant of the patent: **08.11.2017**
(21) Application number: 13866674.8
(22) Date of filing: 20.12.2013
(51) Int. Cl.: A61L 2/03, B08B 3/10

(54) **METHOD AND DEVICE FOR CLEANING, DISINFECTING AND STERILIZING MEDICAL AND HYGIENE ARTICLES AND MEDICAL INSTRUMENTS**
VERFAHREN UND VORRICHTUNG ZUR REINIGUNG, DESINFEKTION UND STERILISATION VON MEDIZINISCHEN UND HYGIENEARTIKELN SOWIE MEDIZINISCHEN INSTRUMENTEN
PROCÉDÉ POUR NETTOYER, DÉSINFECTER ET STÉRILISER DES ARTICLES À USAGE MÉDICAL ET HYGIÉNIQUE ET DES INSTRUMENTS MÉDICAUX ET DISPOSITIF POUR LE METTRE EN OEUVRE

(30) Priority: 24.12.2012 RU 2012157428
(43) Date of publication of application: 28.10.2015
(73) Proprietor: Mamaev, Anatoly Ivanovich, g. Tomsk 634061 (RU)
(72) Inventor: MAMAEVA, Vera Aleksandrovna, g. Tomsk, 634061 (RU); MAMAEV, Anatoly Ivanovich, g. Tomsk, 634061 (RU); MAMAEV, Aleksandr Anatolievich, g. Tomsk, 634061 (RU)
(74) Representative: Sloboshanin, Sergej
(86) International application number: PCT/RU2013/001147
(87) International publication number: WO 2014/104940

(56) References cited:
- WO-A1-93/12821
- KR-A- 20020 078 528
- RU-C2- 2 223 789
- RU-C2- 2 423 907
- SU-A1- 1 285 063
- US-A- 4 909 995
- US-A- 4 943 417

## Description

### TECHNICAL FIELD

This invention relates to medicine and more particularly to methods and devices for disinfecting and sterilizing materials and objects, and can be used for the efficient rapid cleaning, disinfecting and sterilizing of medical articles and instruments, including those that have elongated channels, for example endoscopes and dental air-turbine handpieces.

### PRIOR ART

There are known chemical methods of endoscope sterilization that employ solutions of disinfecting and sterilizing substances that affect microflora and destroy it, for example using the composition disclosed in [RU 94027763A].

It is known that plasma may be employed to sterilize infectious objects. The inventions of Patents US 4 943 417 A, US 4 909 995 A, so as WO 93/12821 A1, for instance, illustrate plasma gas chambers designed specifically for sterilizing medical instruments by using a plasma produced by electrical discharges in a geseous atmosphere.

There are known units to perform complex multi-stage treatment of a medical instrument, for e[ample a unit for cleaning, disinfection and sterilization of endoscopes and medical instruments [UA 77051 C2, 2006]. That effect includes preliminary cleaning with water, ultrasonic cleaning, treatment with disinfectants, rinsing, drying and sterilization. Cleaning and disinfection are performed by treating the article with circulating pulse alternating flow of washing and disinfecting liquid and gas bubbles. The unit consists of profiled bath, circulation system to feed washing, disinfecting and rinsing liquid, ultrasonic cleaning system, drying system, sterilization system, and controller.

The downside of this invention consists in the presence of disinfecting liquids (use of chemical disinfection method) that are known to be molecular poisons; those liquids need to be handled with care and call for protective gear and plenum exhaust ventilation. Moreover, such multi-stage cleaning and sterilization process is time-consuming, as far as it requires holding the article in disinfecting liquid for some time, and time is also needed to wash that liquid off the article. Furthermore, extra time is needed for drying and sterilization.

The same flaws are found in the technical solution disclosed in [JP 2007089638 A] that offers a design resolving the problem of endoscope washing/sterilization and in the one mentioned above [RU 94027763A].

There is a known sterilization device [NZ 549214 A] with a foundation and a lid for disinfection of surgical equipment. Next to the foundation there are an internal sterilization compartment and external spill compartment divided by the inner partition that is lower than the main outer foundation wall. Sterilization compartment has the guides for placing a flexible endoscope with cable length capable of stretching outside the unit through the cable outlet. Sterilization compartment and spill compartment are linked by water drains that are connected to them via control unit.

This invention is based on sterilization of external surface of an endoscope and nothing is said about sterilization of its internal surface, while one needs to clean and sterilize both external and internal surfaces of endoscopes.

There is a known technique of using electrochemical processes for sterilization of endoscopes that consists in washing the instruments and materials over with catholyte and anolyte produced by means of electrochemical activation of water solutions obtained using low polarizing voltages, and their delivery to internal and external endoscope surfaces and/or passing of solution through the endoscope channels [RU 2113859, KR 20020078528].

The known apparatus for cleaning and sterilization of endoscopes [RU 2113859, 1998] contains at least one unit for endoscope fixing equipped with accessories for feeding the washing and sterilizing solutions to internal and external endoscope surfaces and accessories for draining the used solutions, as well as a unit for preparation of washing and sterilizing solutions from initial water solution of reagent containing sodium chloride. The unit for preparation of washing and sterilizing solutions has at least one membranous electrochemical cell, the electrode chambers of which are series-connected by flow channel along the path of the initial solution that is being spent. Inlet of the first electrode chamber in the series is connected to the accessory feeding the initial solution, and outlet of the second chamber is connected to the accessories that feed the washing and sterilizing solutions to internal and external endoscope surfaces. The difference is that electrochemical cell is vertical and cylindrical with coaxial electrodes and ceramic diaphragm dividing the inter-electrode space into cathode and anode chambers with inlet in the lower and outlet in the upper parts. Accessory for feeding the initial solution is connected to the inlet of the cathode chamber of the cell, while flow channel connects the outlet of cathode chamber with the inlet of anode chamber, and outlet of anode chamber is connected to the accessories that feed washing and sterilizing solutions to internal and external endoscope surfaces. The unit for preparation of the initial solution is connected to the unit for its feeding and includes the tap water delivery line, as well as a unit for filtering the water fed from heavy metal ions and hardness salts, a mixer unit and a tank with concentrated reagent solution. Tap water delivery line, feeding unit and mixer unit are series-connected, and mixer unit is also connected to the tank with concentrated reagent solution.

Disadvantages of this apparatus and the method it relies upon include low degree of sterilization of the treated surfaces due to low biocide activity of the sterilizing solution; furthermore it is cumbersome and its design is too complex.

There is a known [KR 20020078528] endoscope washing and sterilizing unit with the following components: electrolytic tank producing water for sterilization and washing; platinum group electrodes that are placed in a sequence: cathode, anode, cathode, anode and cathode, in order to increase the efficiency of reaction with the electrolytic tank; power supply providing sufficient electrolytic current in the tank; ventilator that cools down high temperature produced by the power supply; pump that feeds solution to the tank for reaction; pump that brings sterilizing water from the tank for circulation inside and outside the endoscope; induction valve that is automatically blocked when tap water is fed to the tank; automatic caisson valve that maintains uniform concentration of sterilizing water; and adaptive circuit that automatically regulates the whole process. The goal of this invention is to develop a unit for endoscope sterilization that produces water for sterilization containing NaOCl produced through electrode reaction: electrode reaction produces sodium hypochlorite that is a strong oxidizer and performs disinfection of both external and internal sides of an endoscope.

The disadvantage of this sterilizer is the need to cool the sterilization water down using a ventilator, as far as hot sodium hypochlorite solution may have a destructive effect on the endoscope surface.

There is a known method of using pulse electric discharges excited in solutions for cleaning and sterilization of channels of elongated tubular medical articles, in order to form shock waves having destructive effect upon various impurities of internal channel surfaces.

For example, there is a known device for cleaning and sterilization of channels of elongated tubular medical articles disclosed in [RU 2062971, C1] that contains two coaxially mounted electrodes separated by an insulation bushing and connected to the source of electric pulse discharges.

There is also a known device for cleaning and sterilization of channels of elongated tubular articles [RU 2355425, 2009], primarily endoscopes, that contains a central electrode mounted in lengthwise movable insulation bushing, carrying the second electrode, that are connected to the source of electric pulse discharges and are placed in the treated channels filled with pumped working liquid, whereas the central electrode has a through axial opening and is equipped with a flange resting on the insulation bushing end and mounted on the side of the second stick electrode, that is fixed in axial alignment using brackets of the cylindrical current lead surrounding the insulation bushing and flanking the treated channel with guaranteed annular ring. These distinctive features ensure automatic complex treatment, including cleaning and sterilization, of channels of elongated tubular articles by means of longitudinal displacement of the source of pulse discharges with accompanying hydraulic shocks and ultraviolet radiation exerting uniform effect upon internal surface of the treated endoscope.

The two alternatives described above are characterized by design complexity: it is inconvenient that the source of pulse discharges needs to be displaced longitudinally along the endoscope together with the source of UV radiation; it is also possible that the effect may be insufficient for cleaning and sterilization of an endoscope. No information is given regarding external endoscope sterilization. Moreover, this method cannot be applied to channels of medical instruments with 1-4 mm diameter.

There is a known method and device for endoscope sterilization [JP2004057324 A] using direct electrochemical reaction, i.e. actual passing of current through microorganisms. An endoscope is immersed in solution, and electric current is passed between a positive and a negative electrodes, between which one either places an endoscope without direct contact with electrodes or brings it in contact with one of the electrodes, thus sterilizing the endoscope. According to the authors, the suggested sterilization method allows sterilizing endoscopes without using expensive medicinal solutions and enable one to avoid the flaws of methods based on sterilization in autoclaves.

One of its disadvantages is low process intensity, as far as its effect is based on the source of direct current. In the meantime the source [JP2004057324 A] does not list solutions for reaction or values of current and voltage under which microorganisms can be destroyed.

There are known methods of cleaning and sterilization of metallic and non-metallic instruments using microplasma discharges excited in electroconductive solutions described in the Russian patents No. 2223789, 2126691, 2082435. The essence of these methods consists in the fact that medical articles and instruments are placed in sterilization chamber filled with electrically conductive medium and are subjected to the effect of electric current that is fed from the power source via electrodes placed in the chamber, while microplasma discharges are generated on the electrodes in the chamber.

In the medical instrument sterilization method [RU 2082435 C1, 1997] instruments are placed in the current-conducting medium and are subjected to the effect of microarc discharges created by discharge electrodes connected to the pulse power source, and the treated medical instrument is itself used as one of the electrodes (cathode).

In the method for medial instrument cleaning and sterilization [RU, 2126691 C1, 1999] instruments are placed in the sterilization chamber filled with liquid current-conducting medium and are subjected to the effect of electric current by means of using the instrument itself as one of the electrodes and generating microplasma discharge on the counter electrode.

The disadvantages of the two alternatives described above are impossibility of treating non-metallic materials and instruments and impossibility of treating combined instruments, i.e. metallic instruments with non-metallic components, as far as the treated instrument is used as one of the electrodes, and this would lead to non-metallic components loosing such properties as elasticity and durability.

In the method for cleaning and sterilization of non-metallic materials and instruments [RU Nº2223789 C2, 2004] materials and instruments are placed in the sterilization chamber filled with electrically conductive medium and are subjected to the indirect effect of electric current that is fed from the power source via electrodes placed in the chamber without electroconductive contact of materials and instruments with electrodes, whereas the treated materials and instruments are placed in the immediate proximity of the electrodes and microplasma discharge is generated on at least one of them.

The disadvantage of the alternative described above chosen as a prototype is the fact that it envisages generation of microplasma discharges on only one of the electrodes - either anode or cathode, and the disadvantage of device disclosed in [RU Nº2223789 C2, 2004] is the fact that it does not envisage the possibility of cleaning internal surfaces of elongated medical articles with channels, such as, for example, endoscopes.

### INVENTION DISCLOSURE

The object of the present invention is to develop an efficient method and device that would allow for simultaneous express cleaning, disinfecting and sterilizing of medical articles and instruments.

Another object of this invention is to develop an efficient method and device that would allow for simultaneous express cleaning, disinfecting and sterilizing of a medical instrument that has elongated channels, i.e. sterilization of both external surface of the instrument and internal surface of its channels.

One more object of this invention is to develop an efficient method and device that would allow for simultaneous express cleaning, disinfecting and sterilizing of medical articles and instruments with porous surfaces.

The object set is achieved by the technical features of the independent claims. As in the known method, in the claimed method for cleaning, disinfecting and sterilizing of medical and hygiene articles and medical instruments, the treated articles or instruments are placed in the chamber filled with electrically conductive medium and are subjected to the indirect effect of electric current supplied to electrodes, said electrodes being pre-positioned in the chamber and connected to the power supply without electrically conductive contact between the medical instruments and the electrodes. In order to ensure intensive indirect effect of electric current upon the treated medical articles or instruments, they are placed in the immediate proximity of the electrodes: at least one anode and/or at least one cathode, whereas electric current effect is provided upon condition of simultaneous microplasma discharges generation on all electrodes.

In addition to that, electrically conductive medium is circulated in the chamber.

In order to ensure cyclic effect of electrically conductive medium upon the treated medical article or instrument, its pulsing feed is ensured.

Preferably, in order to intensify the process of cleaning and sterilizing, electrically conductive medium is circulated and additionally activated with microplasma discharges in at least one additional chamber.

In addition, the treated articles or medical instruments are subjected to the effect of 'step voltage'-voltage drop generated by two electrodes, one of which is placed in the chamber next to the inlet of the activated electrically conductive medium, while the second electrode is placed at the outlet of electrically conductive medium from the chamber.

Step voltage is understood as voltage drop per unit of length in the space between two electrodes, through which electric current is passed.

In addition, the treated articles or instruments made of metals are subjected to the direct effect of electric current connecting it to one of the electrodes - anode or cathode.

Moreover, a microplasma discharges are generated on electrodes, namely multiple alternating anodes and cathodes in the form of metallic plates of the same size that are placed uniformly throughout the volume of the chamber.

The electrodes represent the single array formed by a multitude of alternating anodes and cathodes insulated from each other.

There is a strong preference for electrodes, both anodes and cathodes that are made of aluminum, titanium, zirconium or alloys thereof.

Furthermore, the above-mentioned treated articles or medical instrumets are placed in the chamber in the guides above or between the electrodes - anode and cathode, or in the bulk of the above-mentioned electrode array.

Furthermore, non-toxic conductive water solutions are used as electrically conductive medium.

Microplasma discharges are generated at voltage of up to 1000 V, preferably 400-600 V, using anode-cathode mode with current density of up to 500 A/dm², preferably 250-400 A/dm², or the mode with sinusoidal voltage and current density of up to 50 A/dm², preferably 30-40 A/dm².

Furthermore, the process of cleaning disinfecting and sterilizing is carried out at electrically conductive medium temperature of no more than 80⁰C (preferably mo more than 50⁰C).

The object set is also achieved as follows: as in the known method, in the claimed method for cleaning, disinfecting and sterilizing of medical articles and instruments with elongated channels, primarily endoscopes and dental air-turbine handpieces, the treated articles or instruments are placed in the chamber filled with electrically conductive medium and are subjected to the indirect effect of electric current that is fed to the electrodes placed in the chamber and connected to the power supply without electroconductive contact between medical instruments and the electrodes.

Novelty consists in the fact that in order to ensure intensive indirect effect of electric current upon above-mentioned articles and instruments, they are placed in the immediate proximity of electrodes: at least one anode and at least one cathode, whereas electric current effect is provided upon condition of microplasma discharge generation on all electrodes simultaneously; in addition to that, electrically conductive medium is circulated in the chamber and/or instrument channels.

Furthermore, in order to ensure cyclic effect of electrically conductive medium on the treated articles or instruments, its pulsing feed is provided.

In addition, electrically conductive medium that consists of active water-gas mix in the form of foam is circulated through the instrument channels and activated with microplasma discharges in the additional chamber.

Preferably, medical instruments with channels are additionally subjected to the effect of 'step voltage' - voltage drop (voltage difference) generated by two additional electrodes, one of which is placed next to the channel inlets, and the second one is placed next to the outlet from the channels, or inlet and outlet of each channel.

Preferably, microplasma discharge is generated on the electrodes that are multiple alternating anodes and cathodes in the form of metallic plates of the same size uniformly distributed throughout the chamber volume.

Furthermore, the electrodes are insulated from each other with dielectric material and form the single electrode array.

Preferably, the electrodes, both anodes and cathodes, are made of aluminum, titanium, zirconium or alloys thereof.

Furthermore, the above-mentioned treated instruments are placed in the chamber on the guides between the electrodes: anode and cathode, or in the volume of the above-mentioned electrode array.

Furthermore, non-toxic conductive water solutions are used as electrically conductive medium.

Microplasma discharges are generated at voltage of up to 1000 V, preferably 400-600 V, using anode-cathode mode with current density of up to 500 A/dm², preferably 250-400 A/dm², or the mode with sinusoidal voltage and current density of up to 50 A/dm², preferably 30-40 A/dm².

Furthermore, the process of cleaning and sterilization is carried out at electrically conductive medium temperature of no more than 80⁰C, preferably mo more than 50⁰C.

Another object set is achieved as follows: as in the known method, in the claimed method for cleaning, disinfection and sterilization of medical and hygiene articles and medical instruments with porous structure, primarily implants, the treated articles or instruments are placed in the chamber filled with electrically conductive medium and are subjected to the indirect effect of electric current fed to the electrodes placed in the chamber and connected to the power supply.

Novelty consists in the fact that in order to ensure intensive indirect effect of electric current upon the treated articles or instruments, they are placed in the immediate proximity of the electrodes: at least one anode and/or at least one cathode, whereas electric current effect is provided upon condition of simultaneous microplasma discharge generation on all electrodes and under conditions of reduced pressure equal to the partial pressure of electrically conductive medium vapors.

In addition to that, electrically conductive medium is circulated in the chamber.

In order to ensure the cyclic effect of electrically conductive medium upon the treated article or instrument, its pulsing feed is provided.

Furthermore, in order to intensify the process of cleaning and sterilization, electrically conductive medium is circulated and additionally activated with microplasma discharges.

In addition, the treated articles or instruments are subjected to the effect of step voltage -voltage drop generated by two electrodes, one of which is placed in the chamber next to the inlet of the activated electrically conductive medium, while the second electrode is placed at the outlet of electrically conductive medium from the chamber.

In addition, the treated articles or instruments made of metals are subjected to the direct effect of electric current connecting it to one of the electrodes - anode or cathode.

Furthermore, a microplasma discharges are generated on the electrodes, namely multiple alternating anodes and cathodes in the form of metallic plates of the same size that are placed uniformly throughout the volume of the chamber.

The electrodes represent the single array formed by a multitude of alternating anodes and cathodes insulated from each other.

Preferably, the electrodes, both anodes and cathodes, are made from aluminum, titanium, zirconium or alloys thereof.

Furthermore, the above-mentioned treated instruments are placed in the chamber on the guides between the electrodes: anode and cathode, or in the volume of the above-mentioned electrode array.

Furthermore, non-toxic conductive water solutions are used as electrically conductive medium.

Microplasma discharges are generated at voltage of up to 1000 V, preferably 400-600 V, using anode-cathode mode with current density of up to 500 A/dm², preferably 250-400 A/dm² or the mode with sinusoidal voltage and current density of up to 50 A/dm², preferably 30-40 A/dm².

Furthermore, the process of cleaning disinfection and sterilization is carried out at electrically conductive medium temperature of no more than 80⁰C, preferably mo more than 50⁰C.

The object set is also achieved by the fact that claimed device for cleaning, disinfecting and sterilizing of medical and hygiene articles and medical instruments contains at least one main microplasma chamber designed for placement of the treated articles and instruments and electrically conductive medium, equipped with electrodes that are connected to the pulse power supply, as well as the system of electrically conductive medium circulation ensuring delivery of electrically conductive medium to the said chamber and its drainage from the chamber.

Preferably, the device can have at least one additional microplasma chamber designed for preparation (additional activation) of electrically conductive medium, where electrodes are placed to generate microplasma discharges.

The circulation system contains at least one filter placed at the outlet of the main microplasma chamber for mechanical cleaning of electrically conductive medium, the system of pumps and valves to feed the refined electrically conductive medium to the main microplasma chamber or to the additional microplasma chamber - for its activation with microplasma discharges.

Preferably, the device meant for cleaning, disinfection and sterilization of medical instrumets with elongated channels has at least one additional separate microplasma chamber for each channel, designed for preparation (additional activation) of electrically conductive medium, where electrodes are placed to generate microplasma discharges.

The circulation system meant for cleaning, disinfection and sterilization of medical articles and instruments with elongated channels contains at least one filter placed at the outlet of the main microplasma chamber for mechanical cleaning of electrically conductive medium, the system of pumps and valves to feed the refined electrically conductive medium to the main microplasma chamber and to the channels of articles of instruments, and/or to the additional microplasma chamber - for its activation with microplasma discharges.

Additional microplasma chamber is connected either to the first pulse power supply meant to feed the electrodes of the main microplasma chamber or to the second separate pulse supply of power.

Furthermore, in order to create 'step voltage' - voltage drop (application of additional potential difference) in the main microplasma chamber and intensify the cleaning, disinfection and sterilization processes, there are additional electrodes either at the inlet and outlet of the chamber, or at the inlet and outlet of each channel in case of treating the instrument with channels, in order to intensify the cleaning, disinfecting and sterilizing of each channel of the treated instrument.

Furthermore, it also contains the third power supply designed to generate step voltage - voltage drop (application of additional potential difference) in the chamber or in each channel to which the above-mentioned additional electrodes are connected.

The suggested device for cleaning, disinfecting and sterilizing of medical articles and instruments with porous surface, primarily implants, has additional means to hermetically seal the main microplasma chamber and means to create reduced pressure in it.

Furthermore, the device is additionally equipped with computer control system.

Furthermore, the main microplasma chamber has a housing with a multitude of electrodes of the same size, i.e. alternating anodes and cathodes shaped as plates and insulated from each other with dielectric material, thus forming the single array.

There are guides in the electrode array or above it, designed for placement of the treated medical instrument.

Furthermore, the main microplasma chamber has a horizontally oriented housing, where elongated electrodes (anode and cathode) are located in the immediate proximity to each other, designed for placement of the treated elongated medical instruments, including instruments with channels.

Furthermore, the above-mentioned chamber has an outlet on its end for the medical instruments with channels to extend outside the chamber, when necessary.

Furthermore, the main microplasma chamber has an insulated housing, where electrodes are located: anode and cathode, one of which is shaped as a hollow cylinder, inside which there is a vertically oriented container with guides to place the treated medical instrument, and the second electrode represents at least one vertically oriented cylindrical electrode installed in the container.

Furthermore, the main microplasma chamber has an insulated housing, where electrodes are located: anode and cathode, one of which is shaped as a hollow cylinder, the walls of which are made of multiple plates: alternating anodes and cathodes separated by the dielectric material, whereas inside said hollow cylinder there is a vertically oriented container with guides for placement of the treated medical instrument.

Furthermore, container in the vertical chamber is removable, i.e. it can be easily put in or taken out through the upper part of the cylinder.

The distance between electrodes in the chamber must be sufficient for emergence of the microplasma process at least on one of the electrodes and must not result in disruptive discharge between the electrodes.

Preferred distance is 5-20 mm. Increase in the distance leads to reduced efficiency of solution treatment, while decrease in the distance results in additional heating of the solution, and when the temperature of 50 ⁰C is exceeded this is a factor having a negative effect upon the treated instrument.

Furthermore, additional microplasma chamber has at least two electrodes located in the chamber in such a way that they are both washed over by the pumped solution (electrically conductive medium).

It is possible to shape the above-mentioned additional chamber as two tubular electrodes, for example fittings, also located at the distance of 5-20 mm from each other and connected to each other with silicone tubes.

In the latter case the electrodes are shaped as tubular electrodes (fitting pieces), though the shape does not actually matter.

It is possible to produce the above-mentioned additional chamber in the form of a housing made of non-electroconductive material with electrodes and means to fix current-conducting wires and tubes to feed and discharge the solution.

Furthermore, the main and additional microplasma chambers are equipped with electrodes that are made of valve metals as aluminum, titanium, zirconium or their alloys with oxide coating on their surface or without a coating.

It is preferable to use compound as a dielectric material.

### BRIEF DESCRIPTION OF DRAWINGS

Fig. 1 presents top view (a) and sectional view (b) of the main horizontal microplasma chamber with horizontally oriented housing, where elongated electrodes (anode and cathode) are located in the immediate proximity to each other, and between the electrodes there are guides meant for placing the treated articles and medical instruments (both small and elongated ones, including instruments with channels).
Fig. 2 presents the main microplasma chamber equipped with the single array of electrodes, alternating anodes and cathodes, that are covered with compound (horizontal microplasma chamber), whereas there are guides in the array designed for placing the treated medical instrument.
Fig. 3 presents sectional view (a) and top view (b) of the main vertical microplasma chamber for cleaning disinfecting and sterilizing of medical instruments, where one electrode is a hollow vertically oriented cylinder, inside which the second one is located in the center - a vertically oriented stick-shaped electrode; a tank with guides for placing the medical instrument is located around the second electrode and coaxially in relation to the cylinder.
Fig. 4 presents the layout of the device for cleaning, disinfection and sterilization of medical instruments with channels, that has one power supply and the main horizontal microplasma chamber given in Fig. 2.
Fig. 5 presents the layout of the suggested device for cleaning, disinfection and sterilization of medical instruments with channels, that has three power supply, the main horizontal microplasma chamber (Fig. 2) and one additional microplasma chamber.
Fig. 6 presents the layout of the device that has three power supply, the main horizontal microplasma chamber (Fig. 2) and additional chambers placed in front of each channel for solution activation.
Fig. 7 presents the layout of the device that has one power supply, the main horizontal microplasma chamber (Fig. 2) and additional chambers placed in front of each channel for solution activation.
Fig. 8 presents the diagram of the device operation when using three power supply.
Fig. 9 presents medical articles and instruments made of different materials: a - medical articles (instruments) contaminated with blood prior to microplasma treatment; b - after microplasma treatment.
Fig. 10 presents analysis results of the medical instrument subjected to microplasma treatment and analyzed using Clean-TraceTMProtein Test.

### DETAILED DESCRIPTION OF PREFERRED EMBODIMENTS

In the claimed method the treated articles, for example medical instruments, are put into the special solution, namely electrically conductive medium, where it is possible to initiate microplasma processes and electrochemical reactions and where electrodes are also placed, on which under certain conditions and modes microplasma discharges are generated (on anode and cathode simultaneously). High pressure pulses, shock waves and hydrodynamic flows are generated in the solution during electrical breakdown; breakdown is accompanied with light emission of the low-temperature plasma discharge, molecules are ionized and dissociated, free radicals are formed, ultraviolet and ultrasonic radiations are generated in addition to the pulsing electromagnetic field. Thus, a medical instrument is subjected to complex physical and chemical effect. Low temperature plasma is capable of inactivating various microorganisms: bacteria (including spore forms), viruses, fungi and organic matter.

The method is based on techniques of cleaning and sterilization using microplasma discharges excited in electrically conductive medium described earlier in the Russian patents No. 2223789, 2126691, 2082435.

The difference of the claimed method from the known ones described above is the fact that, first of all, medical instruments are subjected to the indirect effect of electric current upon condition of microplasma discharge generation in the main microplasma chamber (chamber meant for placement of the treated instruments) on all electrodes simultaneously: on anodes and cathodes; and second, the fact that sterilizing solution (activated electrically conductive medium) is circulated in the chamber.

In the claimed method the sterilizing solution (activated electrically conductive medium) is circulated during medical instrument treatment along the surfaces of medical instruments (both external and internal). It is claimed to additionally activate the solution (electrically conductive medium), before passing it through, under the effect of microplasma process not only in the main microplasma chamber, but also in the additional microplasma chambers and then pump it under pressure through the main microplasma chamber, including through the instrument channels.

One more additional factor is envisaged in the present invention for non-metallic medical instruments: indirect effect of electric current by means of step voltage. In case of instruments with elongated shape and channels, primarily endoscopes and dental air-turbine handpieces, voltage difference (step voltage) is generated in the channels of the treated instrument and it causes additional effect killing microbes and bacteria.

Sterilization is carried out at temperatures not exceeding 80⁰C, preferably at temperatures not exceeding 50⁰C, i.e. temperatures not modifying properties of the instrument surface.

Furthermore, the present invention envisages one more additional factor for metallic medical articles and instruments, namely direct effect of electric current.

In case of medical articles and instruments with pores the present invention also envisages generation of reduced pressure equal to partial pressure of electrically conductive medium vapors in the main microplasma chamber, which is necessary for removal of air from the pores. Alternation of the reduced pressure over electrically conductive medium and the medium circulation pressure allows for reliable cleaning, disinfection and sterilization of the medical instrument pores.

Healthcare faces the problem of sterilization of porous materials, as far as it is rather difficult to remove air from the pores. In order to resolve that problem, it is claimed to connect the main microplasma chamber to the vacuum-pumping system and combine alternating pressure (i.e. change the value of pressure cyclically) in the microplasma system.

The claimed device contains the main microplasma chamber (MP1) 1 for placement of the treated medical instrument, i.e. in the present invention the main microplasma chamber (MP1) is understood as a chamber, where one places medical articles or instruments that are to be cleaned, disinfected and sterilized. MP1 (main microplasma chamber) is equipped with electrodes - cathode and anode, on which the microplasma process is simultaneously generated. As a result of electrochemical reactions and microplasma processes in the chamber, gas is emitted, near-electrode electrolyte is alkalized and acidified, whereas activation and ionization of the solution is accompanied by formation of active particles - radicals. Meanwhile as a result of gas emission and washing over by the activated solution (electrically conductive medium), external surface of the instrument is cleaned, disinfected and sterilized. Solution activated by above-mentioned processes is circulated through the chamber by the pump and in case of treatment of instruments with channels - through the instrument channels, cleaning, disinfecting and sterilizing them.

The present invention envisages several design options for the main microplasma chamber, including an option for treating instruments with channels.

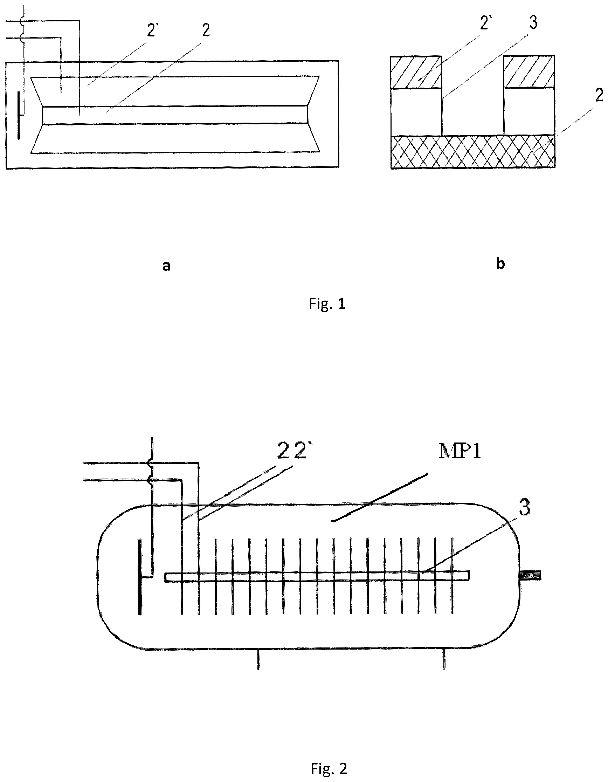

In one of the options (Fig. 1) the main microplasma chamber MP1 (horizontal chamber) is equipped with electrodes 2 and 2'- cathodes and anodes, to which the power supply (PS1) feeds voltage in the form of anode-cathode pulses for generation of the microplasma process on the electrodes. There is a guiding profile (guides 3) between these electrodes, where medical instruments are put, for example instruments with channels (flexible endoscope, air-turbine handpiece).

Electrodes can be made up of multiple aluminum or titanium plates of the same size (Fig. 2) and uniformly distributed throughout the volume of the microplasma chamber 1, separated by dielectric material and forming the single electrode array. Such design allows electrodes to operate simultaneously in the microplasma mode; electrodes can be in use for a rather long time (10 years). Besides, as a result of the uniform distribution of electrodes microplasma processes occur uniformly throughout the whole chamber, and electrolyte is uniformly heated without exceeding the required temperature of 50⁰C. A medical instrument with channels, for example an endoscope, placed on guides, is washed over by the activated solution from all sides. Microplasma processes and temperature mode in the course of 3-5 minutes enable one to uniformly and simultaneously clean, disinfect and sterilize the whole non-metallic surface of an endoscope without modifying the properties of its surface (elasticity, integrity).

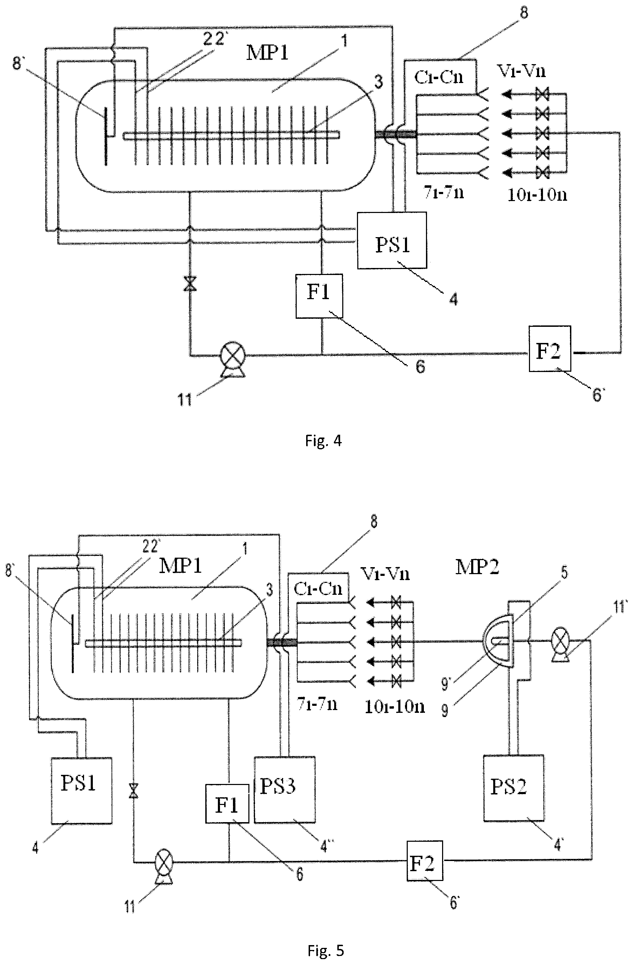

The device (Fig. 4) has at least one circuit of electrolyte circulation for cleaning, disinfection and sterilization of medical instrument channels (C₁-Cₙ). Activated solution is pumped under pressure from the main microplasma chamber (MP1) through the instrument channels. Solution is cleaned from mechanical admixtures at the filter (F1) installed at the outlet from the MP1.

In order to intensify the cleaning, disinfection and sterilization of the medical instrument channels (C₁-Cₙ), the device can contain an additional circuit of activated solution circulation. Such circuit has additional microplasma chambers (MP2) 5, where microplasma process also takes place and additionally activates the solution (electrically conductive medium) that has been previously activated in MP1. Through filter (F1) 6 the solution is taken from the main microplasma chamber (MP1) 1 and to the auxiliary microplasma chamber (MP2) 5. In additional microplasma chamber (MP2) 5 solution that has gone through the filter (F1) 6 is cleaned and activated, which allows for multiple use of the same solution.

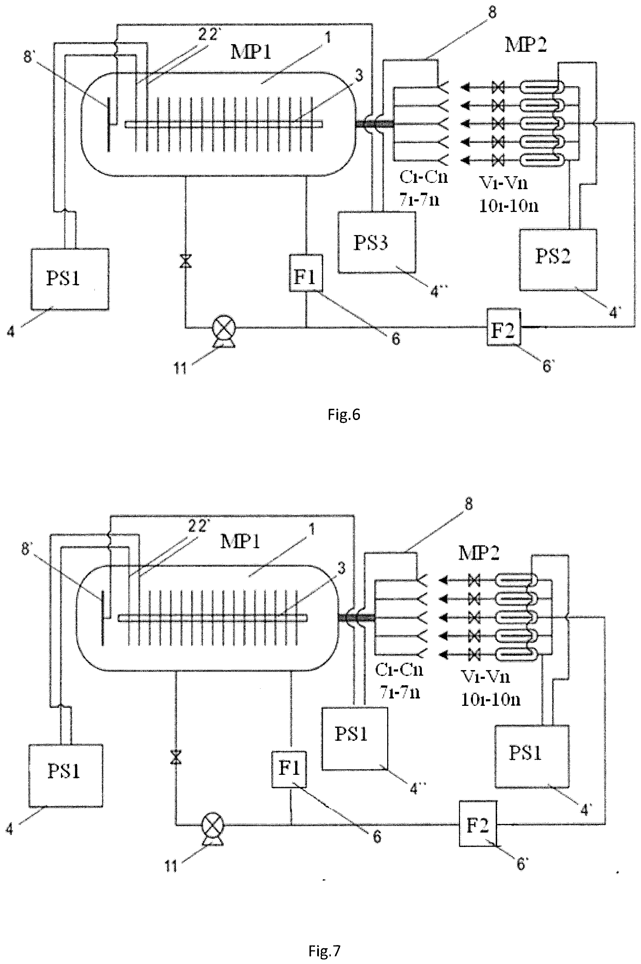

Therefore, mechanically cleaned activated solution that went under pressure through the filter in the device, after leaving the main microplasma chamber gets into and is treated in at least one additional chamber (MP2) 5 or several additional microplasma chambers (depending on the number of channels) (Fig. 6 and 7).

Microplasma discharges initiated in the additional chamber (MP2) 5 next to the inlet of the instrument channels lead to the following effects:
- Solution is activated under the influence of microplasma discharges. A lot of active radicals emerge in it. Radicals, active chemical compounds or ions, can be short-living and long-living. Short-living radicals possess higher free energy, but quickly enter into reactions and get neutralized. Activation right before the entry of radicals into the channel results in radicals entering into reaction inside the channels and ensuring disinfection and sterilization.
- The second effect: during operation of the additional microplasma chamber (MP2) 5 foamy gas-liquid mixture emerges and is passed through the channels. Emerging foam has a cleaning effect, just like the soap suds, which accounts for cleaning and sterilizing effect.
- The third effect: the foam passing through the channels has smaller cross-section. Resistance of the channel with foam is higher than resistance of the solution (electrically conductive medium), and when pulse current of a certain value is passed, the step voltage drop is higher. As far as emerging foam is a surface acting agent, it is localized on the surface and bacteria are destroyed due to high step voltage drop (voltage difference).
- The fourth aspect of the effect of microplasma discharges upon the solution is that emerging radicals and microplasma discharge itself destroy proteins making it into the solution after its primary passing through the instrument channels and along the instrument. Protein destruction leads to the destruction of material that makes up viruses, bacteria, fungi and spores, which creates the cleaning effect for the pumped solution.

The present invention also envisages one more factor that has additional effect, namely step voltage killing off microbes and bacteria. For that purpose the device designed for cleaning and sterilization of instruments with channels can contain two electrodes, one of which is located next to the inlet of the instrument channel and the second - on the other side, near the channel outlet. Electrodes are connected to the pulse power supply. As far as active water-gas mixture (activated solution) forms as foam in the additional microplasma chamber (MP2) 5, its passing under pressure through the channels results in the foam moistening the inner surface of the channel, decreasing cross-section and simultaneously increasing resistance and hence the effect of step voltage drop, the effect of which is described above.

Thus, internal channel surfaces are subjected to the additional effect of electric current (step voltage), active part of the sterilizing solution (hydrogen-oxygen part) and activated electrolyte solution due to the passing of liquid.

When gas-liquid mixture is circulated in the main microplasma chamber, the surface layer of the instrument is also subjected to the effect of activated ionized solution (electrically conductive medium).

Thus, unlike the known alternatives (RU No. 2223789, 2126691, 2082435), the claimed device allows arranging electric current passage not only over the surface of the treated instrument, but also during the passage of the sterilizing solution (electrically conductive medium) along the channels (cavities) of articles and instruments.

Microplasma discharges are generated at voltage values of up to 1000 V (preferably 400-600 V) using anode-cathode mode with current density of up to 500 A/dm² (preferably 250-400 A/dm²) or mode with sinusoidal voltage with current density of up to 50 A/dm² (preferably 30-40 A/dm²).

Non-toxic electrically conductive water solutions are preferable.

The device envisages periodic foam removal in the course of operation by way of increasing the solution level and spilling the foam over the upper edge to the system for contaminated solution removal, as far as in the course of device operation proteins are destroyed into secondary and tertiary structures that under the effect of emitted gas gather on the surface in the form of foam.

The most preferable configurations for cleaning, disinfection and sterilization of medical articles (instruments) with channels are device options given in Fig. 5 or Fig. 6: with three power supply and the main microplasma chamber (MP1) 1 (Fig. 2) equipped with the single array of electrodes, alternating anodes and cathodes 2, 2' that are covered with compound. In the array of electrodes 2 and 2' there are guides 3 to place the treated instrument and fill with electrically conductive medium. Electrodes 2, 2' are connected to the first pulse power supply (PS1) 4. Additional microplasma chamber (MP2) 5 (Fig. 5) is designed for activation of solution (electrically conductive medium) coming from the main chamber (MP1) 1 after its removing mechanical admixtures at the filter (F1) 6; electrodes of this chamber are connected to the second power supply (PS2) 4'.

In order to enhance the cleaning, disinfection and sterilization effect, at the inlet of each channel (Fig. 6 or Fig. 7) there can be a separate additional microplasma chamber (MP2) 7₁-7ₙ, (where n is the number of medical instrument channels), electrodes of which are connected to the pulse power source (PS2) 4'.

In another option of device design the channels themselves can act as chambers (MP2) 7₁-7ₙ.

In order to create step voltage - voltage drop in each channel, i.e. to ensure the supply of high voltage to the instrument channel, the device can be equipped with two additional electrodes 8 and 8' that are located as follows: one at the channel inlet and one at the outlet, without touching the channels. The electrodes are connected to the third high voltage power supply (PS3) 4".

Additional microplasma chamber (MP2) 5 has two electrodes (9 and 9') that are washed over by the pumped solution (electrically conductive medium). In practical terms additional microplasma chamber can be made of two fittings connected by hoses. Fittings are connected to the poles of the power source (PS2) 4'.

Pulse voltage leads to additional activation of solution that takes place due to microplasma discharges on electrodes 9 and 9', and the use of electrodes 8 and 8' creates step voltage - voltage drop along the channels enhancing the cleaning, disinfection and sterilization effect.

The circulation system is equipped with valves (V₁ - Vₙ) 10₁-10ₙ feeding the activated electrically conductive medium from the additional chamber (MP2) 5 to the channels of the treated instrument 7₁-7ₙ (Fig. 6).

The system of solution (electrically conductive medium) circulation comprises filters (F1) 6 and filter (F2) 6', the main (MP1) 1 and additional microplasma chambers (MP2) 5, as well as pumps 11 and 11' for passing the activated solution under pressure through the channels of the treated instrument 7₁-7ₙ.

Chamber (MP1) 1 has an outlet on its end (not shown) that makes it possible to extend the treated instrument channels 7₁-7ₙ outside the chamber.

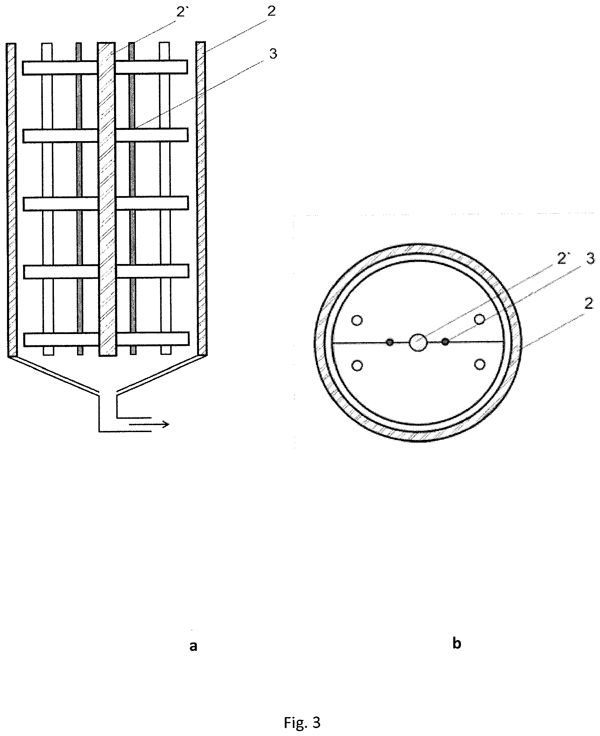

Fig. 3 presents a design option of the main vertical microplasma chamber 1. One chamber electrode is shaped as a hollow cylinder 2' (that can act as housing in one of design options), inside which in the center there is another vertically oriented stick-shaped electrode 2, and there is a container with guides 3 for placing a medical instrument that is located coaxially in relation to the cylinder around the second electrode. Container 12 is removable, dismountable, and has open-framed structure to facilitate placement of a medical instrument between the electrodes and solution delivery to it. Fig. 3 presents container design for articles, such as flexible endoscope. For dental air-turbine handpieces the design of container 12 can be modified depending on the type of air-turbine handpiece, provided that the instrument is placed between the electrodes.

Electrodes in the microplasma chambers (MP1, MP2) 1 and 5 are located in such a way that the distance between them is from 10 to 5 mm and sterilizing solution can be freely pumped through the space between the electrodes.

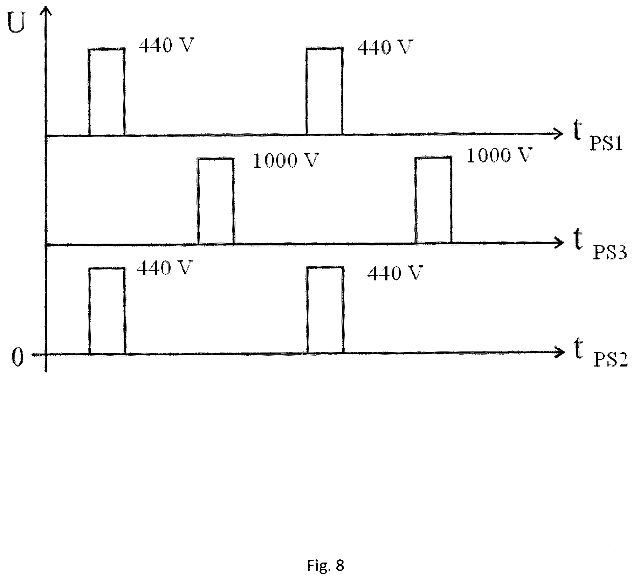

Power supplies (PS1, PS2) 4 and 4' have close technical characteristics. Connection of supplies and order of pulses for them are arbitrary and do not have to be coordinated. In the simplest case one can use one power supply (PS1) 4 (Fig. 4) with parallel connection of electrodes of the chambers (MP1 and MP2) 1 and 5. Power supply (PS3) 4" has characteristics different from power supply (PS1) 4 and (PS2) 4'. As far as power supply have different characteristics and are pulse supplies, their operation needs to be coordinated. Fig. 8 presents a diagram of joint operation of the power supplies. Power supply (PS3) 4" ensuring the passage of current along the channel (creating step voltage) is started in the interval between pulses of the supplies (PS1 and PS2) 4 and 4'.

The device has computer control system (not shown in the figures) with a program for cleaning, disinfecting and sterilizing of internal channels and surfaces of the treated instruments. All operations are automatic. After all procedures are complete, an indicator lights up to inform that the process is complete. Computer system also controls the starting order of pumps, power supplies and preparation of the medical article (instrument) before use. In the present invention the instrument preparation is understood as the presence of blowdown system (not shown in the figures) of the instrument and channels with sterile warm air that dries the instrument from the solution - all this being within the single device.

The cycle of cleaning, disinfecting and sterilizing consists of the following stages when using three pulse power sources.

Control objects:
1. Order, time and conditions of power supply operation (PS1, PS2, PS3);
2. Order, time of pump operation (11 and 11').

Control:
1. Duration of filter operation (F1 and F2);
2. Service life of electrically conductive medium and washing solutions;
3. Sterility control;
4. Control of working capacity of electrically conductive medium: pH of electrically conductive medium, electroconductivity.
5. Control of power source parameters:
   - Power supply (PS1) 4:
      - Output voltage up to 600 V:
      - Currents up to 1000 A;
      - Duration up to 200 microseconds;
      - Frequency 50 Hz;
   - Power supply (PS2) 4':
      - Output voltage up to 600 V;
      - Pulse duration up to 200 microseconds;
      - Current up to 80 A.
   - Power supply (PS3) 4":
      - Output voltage up to 1000 V;
      - Current depending on the maximum channel cross-section up to 8 A.

Medical instrument is prepared using the systems for drying channels with heated (no more than 50°C) air that has undergone ultraviolet sterilization.

The instrument is dried by channel blowdown with sterile air and air-blast cleaning on the outside. The air going through the channels removes remaining moisture and prepares the instrument for use. Other procedures of instrument use are standard.

Before using the instrument, electrically conductive medium from the main microplasma chamber is drained off. Valves V₁-Vn to channels closed. Air is let into chamber 1.

The system for instrument preparation for use feeds air to the channels, blowing and pushing out the remaining liquid and drying the channels. After the result is achieved (the instrument is dry and sterile), the system switches off and control system (not shown) displays the signal - the instrument is sterile and ready for use.

Algorithm of said treatment can look as follows:
- Infrared heating of air;
- Ultraviolet sterilization of air;
- Air blow through channels C₁-Cn of the treated medical instrument.

Examples of analysis results for cleaning, disinfecting and sterilizing of various medical instruments based on the claimed method.

### Example 1. Analysis of durability of different medical materials in case of microplasma treatment in the claimed device.

The device with one power source (Fig. 4) and one main horizontal microplasma chamber (Fig. 2) was used for cleaning, disinfecting and sterilization, and microplasma discharges were generated on all electrodes.

Power supply operates off the electricity mains 10-13 A, 220-240 V AC.

Operation mode of pulse power supply: microplasma discharges on the electrodes are generated at voltage of 600 V using pulse anode-cathode mode with current density of 500 A/dm².

Non-toxic water-based borate-phosphate solution (composition is given in table 1) was used as electrically conductive medium (solution for cleaning and sterilization).

**Table 1. Compositions of electrically conductive medium for microplasma treatment**

| No. | Composition | Concentration g/l |
|---|---|---|
| 1 | sodium tetraborate | 30 |
| | sodium gidroortofosfat | 40 |
| 2 | sodium bicarbonate | 10 |
| | ethyl alcohol | 2 |
| 3 | sodium hydroxide | 2 |
| | ethyl alcohol | 2 |

It is known that medical professionals use medical instruments made of metallic materials of different composition, including those that have different deposited coatings, and instrument made of non-metallic materials (silicone tubes, rubber gaskets etc.). In order to demonstrate the possibility and advantages of microplasma treatment for cleaning, disinfection and sterilization of medical instruments, one needs to perform the treatment at least 3,000 times and make sure that composition, structure and properties of medical instrument materials remain the same.

In order to assess the ability of microplasma method not to change the composition, structure and properties of medical instrument materials, studies were carried out to evaluate the effect of microplasma treatment upon different materials for 120 seconds with a 120-second interval between treatments in the course of 20 days. Temperature of electrically conductive medium was under control in the course of treatment: temperature did not exceed 50 ⁰C during the whole treatment process.

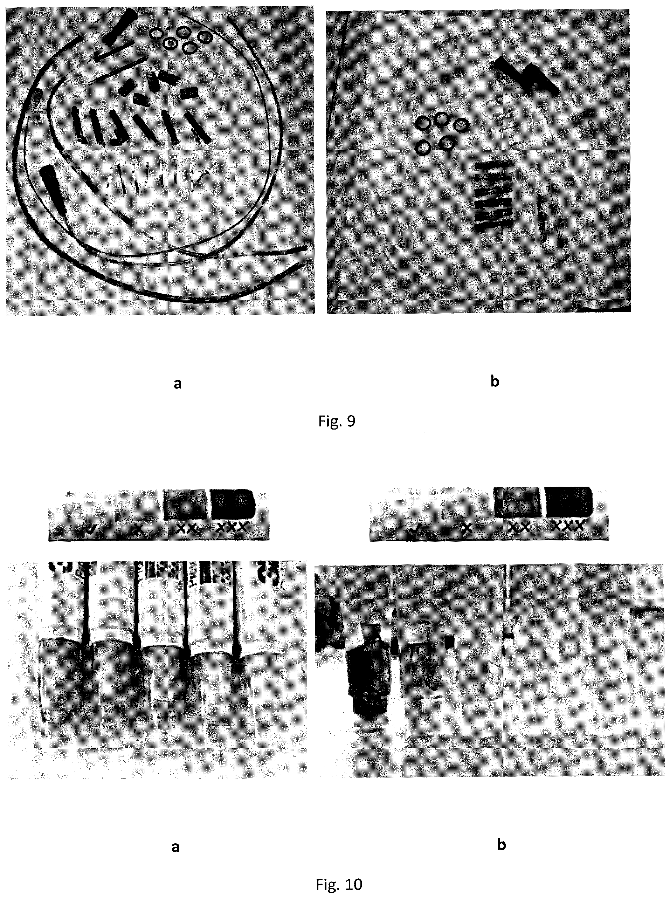

These studies show that in case of microplasma treatment of different materials in the course of 100 hours, materials are neither destroyed nor modified, which is confirmed by visual observation and surface study of the samples after microplasma treatment. Photos of these materials are presented in Fig. 9.

### Example 2. Analysis of efficiency of catheter cleaning, disinfection, sterilization and protein removal using the claimed device.

Examination of the protein destruction process.

The following instrument was selected as a model instrument to evaluate the cleaning, disinfection and sterilization process: Nelaton urinary catheter for men, 400 mm long, produced by SUYUN (China), with 2 mm capillary, external diameter 3 mm, and external surface of 3,768 mm²

Standardized serum solution Biocont C (Certificate: series 11008/121 of 5.11.2008, produced by LLC Agat-Med) was chosen as contaminant. 1 ml portion of it was (using a disposable syringe) into the inner part of catheter (capillary) over electrically conductive medium, contaminating it as well, and this medium was then used in the course of cleaning, disinfection and sterilization.

In order to carry out the study, one used the device with three power supplies (Fig. 5), the main microplasma chamber (Fig. 1) and one additional microplasma chamber 2, the electrodes of which 9 and 9' were connected to PS2, and application of step voltage to the channel (electrodes 8 and 8' were used and they were also connected to PS3).

The volume of solution used for cleaning and sterilization in the device was 600 ml. In the course of operation the device ensures solution circulation at the rate of 6 times per minute. Solution analogous to the one in example 1 was used as electrically conductive medium.

Operation mode of pulse power supplies (PS1) 4 and (PS2) 4': microplasma discharges are generated on the electrodes at voltage of up to 440 V using pulse anode-cathode mode with current density of up to 350 A/dm².

The instrument (Nelaton catheter) was placed into the main microplasma chamber (Fig. 1) and connected to the solution circulation system. Power supply and solution circulation system were started simultaneously and microplasma treatment of the catheter was carried out with activated solution being pumped from the additional microplasma chamber (MP2) 5 through the catheter channel and to the main microplasma chamber (MP1) 1. The time of treatment and its results are given in table 2.

Both the solution itself and the instrument get cleaned, disinfected and sterilized. Contaminants are removed from the instrument surface and its inner part and solution is simultaneously sterilized. Contaminants removed from the instrument surface are destroyed by microplasma discharges and solid waste is accumulated on the filters.

Samples were taken during catheter treatment at 30 second intervals, in order to analyze quantitative and qualitative contaminations.

Analyzed samples were the samples of solution after microplasma treatment and the samples from catheter surface after flushing with distilled water.

Solution samples. Sterile medical 2 ml syringes were used to take solution samples. An individual syringe was used for each measurement. 2 ml samples were taken and placed into clean polyethylene 2 ml containers with lids. Samples were labelled S-10 ('P-10' in Russian), where the letter indicates that this was the solution sample and the figure indicates treatment time.

Washout samples. Catheter was taken out of solution after being treated for a certain period of time. Washout was made from external and internal catheter surfaces using water solution of distilled water. Distilled water washout was put into container for sample and marked W-10 ('C-10' in Russian), where the letter stands for washout, and the figure indicates time of treatment in seconds. Plastic 2 ml containers with lids were used. Each container was only used once.

Blank samples was taken after placing the instrument into solution and stirring for one minute without microplasma treatment.

Blank samples were labelled S-0 and W-0.

Initial catheter contamination was marked W-init and assessed on the basis of norms of surface moistening with contaminating solution (200 ml/m²) with the concentration of contaminating solution 54.3 g/l and catheter surface of 6,280 mm² and was equal to 6.82·10⁻³ g.

In order to assess the cleaning and disinfection effect, one relied on colorimetric microbiuretic method to identify proteins using photoelectric photometer KFK-3. It works as follows: when alkaline copper solution is added to the protein solution, purple coloring appears, and its intensity is proportional to protein concentration. Coloring is caused by the presence of peptide bonds. Sensitivity of this method allows using it in the range of protein concentrations 0.02 g/l- 0.53 g/l. Measurement results were summarized in table 2.

**Table 2. Results of protein concentration measurement in samples at different times of microplasma treatment**

| No. | Treatment time, s | Measurement designation | E, optical density | C, protein concentration in the sample | Cleaning multiplicity |
|---|---|---|---|---|---|
| 0 | 0 | Analysis of initial solution | 0 | less than 0.02 g/l | There is no contamination of initial solution with proteins |
| 1 | 0 | Initial solution contamination S -0 | 0.004 | 3.26 g/l | Experimentally determined concentration |
| 2 | 30 | S -30 | 0 below device sensitivity | less than 0.02 g/l | Cleaning multiplicity - no less than 2.7*10³ times |
| 3 | 60 | S -60 | 0 below device sensitivity | less than 0.02 g/l | Cleaning multiplicity - no less than 2.7*10³ times |
| 4 | 90 | S -90 | 0 below device sensitivity | less than 0.02 g/l | Cleaning multiplicity - no less than 2.7*10³ times |
| 5 | 120 | S -120 | 0 below device sensitivity | less than 0.02 g/l | Cleaning multiplicity - no less than 2.7*10³ times |
| 6 | 150 | S - 150 | 0 below device sensitivity | less than 0.02 g/l | Cleaning multiplicity - no less than 2.7*10³ times |
| 7 | 0 | W - 0 | 0.0072 | 3.41 g/l | |
| 8 | 30 | W - 30 | 0 below device sensitivity | less than 0.02 g/l | Cleaning multiplicity - no less than 2.7*10³ times |
| 9 | 60 | W - 60 | 0 below device sensitivity | less than 0.02 g/l | Cleaning multiplicity - no less than 2.7*10³ times |
| 10 | 90 | W - 90 | 0 below device sensitivity | less than 0.02 g/l | Cleaning multiplicity - no less than 2.7*10³ times |
| 11 | 120 | W - 120 | 0 below device sensitivity | less than 0.02 g/l | Cleaning multiplicity - no less than 2.7*10³ times |
| 12 | 150 | W - 150 | 0 below device sensitivity | less than 0.02 g/l | Cleaning multiplicity - no less than 2.7*10³ times |
| 13 | | W - init | 0.048 | Solution with concentration of 54.3 g/l was injected into catheter | Initial catheter contamination: 6.82 10⁻³ g of protein on the surface of 6,280 mm² |

Table 2 presents multiplicity of cleaning, disinfection and sterilization depending on the treatment time.

### Conclusions:

1. Measurements of trace protein quantities after the cleaning, disinfection and sterilization process in the claimed microplasma device allow talking about reliable cleaning multiplicity of no less than 2.7*10³ times after the 30-second treatment.
2. Microplasma cleaning, disinfecting and sterilizing method reduces the initial concentration of contaminating materials to the detection threshold of the colorimetric method of protein detection based on the microbiuretic technique within the microplasma treatment time equal to 30 seconds.

### Example 3. Analysis of instrument cleaning from blood contamination.

The following articles were used for research: 400 mm Nelaton urinary catheter for men (Netherlands), 500 mm Berocath feeding catheter CH04 with external diameter of 1.3 mm (Germany), as well as two rubber rings, two pieces of silicone tube and three pieces of optic fiber.

Human blood was used as a contaminant and sterile disposable syringe was used to put it inside and outside the catheter and all examined articles; it was dried for an hour and then the instrument was cleaned using the claimed device for microplasma cleaning, disinfection and sterilization.

The volume of solution for cleaning and sterilization in the device was 600 ml. The device ensured solution circulation at the rate of 6 times per minute.

Device layout and modes used were analogous to the ones in example 2.

Three water solutions were used as sterilizing solutions (electroconductive media), and their concentrations and compositions are given in table 1.

In order to assess the efficiency of sample cleaning, experimental studies were carried out as follows.
1. Nelaton male urinary catheter (400 mm long, Suyun, China) contaminated with blood and dried for one hour was placed into the microplasma unit and washed over with electrolyte solution pumped through the internal catheter capillary and outer part of catheter. The first sample of electrolyte solution contaminated with blood and washed off the catheter was taken - sample 1 (W-1).
   Then microplasma treatment of the catheter was carried out. It was placed into the main microplasma chamber 1 and activated solution was pumped through the catheter channel, 2 ml solution samples were taken and put into 2 ml polyethylene containers with lids every 30 seconds in the course of microplasma treatment (samples No. 2, 4, 6, 8, 10) and 1 cm catheter piece was cut off every 30 seconds in the course of microplasma treatment (samples No. 3, 5, 7, 9, 11).
2. Microplasma unit was washed with distilled water, freshly prepared water solution 1 (table 2) made up of borate and phosphate salts was poured, and the sample of this solution was taken - sample 12.
3. Berocath feeding tube (500 mm long, external diameter of 1.3 mm, Germany) contaminated with dry blood was put in, solution was pumped through the catheter channel, sample 13 was taken.
   Then the sample was treated with microplasma and activated solution was pumped through the catheter channel and outer part of catheter, 2 ml electrolyte solution samples were taken into 2 ml polyethylene containers with lids every 30 seconds of microplasma treatment (samples No. 14, 16, 18, 20, 22) and 1 cm catheter piece was cut off every 30 seconds of microplasma treatment (samples No. 15, 17, 19,21,23).
4. Two rubber rings, two pieces of silicone tubes and three pieces of optic fiber were treated in the microplasma unit for 60 seconds. Samples 24-26.

Table 3 lists the sample types and microplasma treatment time for two types of catheters when using sterilization solution No. 1 (table 1).

**Table 3**

| Sample number | Analyzed sample | Stirring time, s | Microplasma treatment | Time of microplasma treatment, s | Total time of microplasma treatment, s |
|---|---|---|---|---|---|
| Nelaton male urinary catheter, 400 mm, Suyun, China | | | | | |
| 1 | solution | 30 | | | |
| 2 | solution | 30 | + | 30 | 30 |
| 3 | catheter | 30 | + | 30 | 30 |
| 4 | solution | 30 | + | 30 | 60 |
| 5 | catheter | 30 | + | 30 | 60 |
| 6 | solution | 30 | + | 30 | 90 |
| 7 | catheter | 30 | + | 30 | 90 |
| 8 | solution | 30 | + | 30 | 120 |
| 9 | catheter | 30 | + | 30 | 120 |
| 10 | solution | 60 | + | 60 | 180 |
| 11 | catheter | 60 | + | 60 | 180 |

| Berocath feeding tube, 500 mm long, Germany | | | | | |
|---|---|---|---|---|---|
| 12 | solution | - | - | - | - |
| 13 | solution after putting the catheter in | 30 | | | |
| 14 | solution | 30 | + | 30 | 30 |
| 15 | catheter | 30 | + | 30 | 30 |
| 16 | solution | 30 | + | 30 | 60 |
| 17 | catheter | 30 | + | 30 | 60 |
| 18 | solution | 30 | + | 30 | 90 |
| 19 | catheter | 30 | + | 30 | 90 |
| 20 | solution | 30 | + | 30 | 120 |
| 21 | catheter | 30 | + | 30 | 120 |
| 22 | solution | 60 | + | 60 | 180 |
| 23 | catheter | 60 | + | 60 | 180 |
| 24 | rubber ring | 60 | + | 60 | 60 |
| 25 | two samples of optic fiber | 60 | + | 60 | 60 |
| 26 | silicone tube | 60 | + | 60 | 60 |

Table 4 lists the sample types and time of microplasma catheter treatment when using bicarbonate electrolyte 2 as a sterilizing solution (table 1): (electrically conductive medium, where it is possible to excite microplasma discharges) (samples No. 30-39).

**Table 4**

| Sample number | | Stirring time, s | Microplasma treatment | Time of microplasma treatment, s | Total time of microplasma treatment, s |
|---|---|---|---|---|---|
| Nelaton male urinary catheter, 400 mm, Suyun, China | | | | | |
| 30 | solution | Stirring, 30 s | | | |
| 31 | solution | 30 | + | 30 | 30 |
| 32 | solution | 30 | + | 30 | 60 |
| 33 | solution | 30 | + | 30 | 90 |
| 34 | solution | 30 | + | 30 | 120 |
| 35 | solution | 30 | + | 30 | 150 |
| 36 | solution | 30 | + | 30 | 180 |
| 37 | solution | 60 | + | 60 | 240 |
| 38 | solution | 60 | + | 60 | 300 |
| 39 | catheter | 60 | + | 60 | 300 |

Table 5 lists the sample types and time of microplasma treatment for two types of articles, solution and catheter, using alkaline electrolyte 3 as a sterilizing solution (table 1) (electrically conductive medium, where it is possible to excite microplasma discharges) (samples No. 40 - 50).

**Table 5**

| Sample number | | Stirring time, s | Microplasma treatment | Time of microplasma treatment, s | Total time of microplasma treatment, s |
|---|---|---|---|---|---|
| 40 | clear solution | | | | |
| 41 | solution | Stirring, 60 s | | | |
| 42 | solution | 30 | + | 30 | 30 |
| 43 | solution | 30 | + | 30 | 60 |
| 44 | solution | 30 | + | 30 | 90 |
| 45 | solution | 30 | + | 30 | 120 |
| 46 | catheter | 30 | + | 30 | 120 |
| 47 | solution | 60 | + | 60 | 180 |
| 48 | solution | 60 | + | 60 | 240 |
| 49 | solution | 60 | + | 60 | 300 |
| 50 | catheter | 60 | + | 60 | 300 |

Solution samples and instrument samples were examined after microplasma treatment at the regular intervals from 0 to 120 seconds. At an interval of 30 seconds 2 ml solution samples and instrument samples were taken.

2 ml aliquots of solution were taken as samples into the special test tubes with lids. Besides, a 10 mm catheter piece was cut off. Catheter piece was put into the leakproof test tube. Samples were assigned numbers. Then the treatment was carried out until the total time of 300 seconds was reached. Samples were taken every 30 seconds.

Solution samples 1-22 were examined at the Tomsk Open Biochemical Laboratory. Test results are given in table 6.

**Table 6**

| Sample No. | Sample composition | Time of microplasma treatment, s | Fe, micromole/l | Biuretic protein g/l | Protein by Lowry method, g/l | Hemoglobin, qualitatively | Na | pH |
|---|---|---|---|---|---|---|---|---|
| 1 | solution | - | 5.4 | 0.7 | 0.68 | pos | 368 | 9.0 |
| 2 | solution | 30 | 3.7 | 0.6 | 0.57 | pos | 354 | 9.1 |
| 4 | solution | 60 | 2.9 | 0.6 | 0.61 | neg | 350 | 9.1 |
| 6 | solution | 90 | 1.8 | 0.6 | 0.57 | neg | 350 | 9.0 |
| 8 | solution | 120 | 1.5 | 0.6 | 0.59 | neg | 350 | 9.0 |
| 10 | solution | 180 | 0.7 | 0.6 | 0.63 | neg | 350 | 9.0 |
| 12 | clear solution | - | 2.8 | 0.3 | 0.04 | neg | 350 | 9.0 |
| 13 | solution | - | 2.7 | 0.1 | 0.05 | neg | 350 | 9.0 |
| 14 | solution | 30 | 2.7 | 0.1 | 0.05 | neg | 350 | 9.0 |
| 16 | solution | 60 | 2.8 | 0.1 | 0.05 | neg | 350 | 9.0 |
| 18 | solution | 90 | 1.9 | 0.2 | 0.05 | neg | 350 | 9.0 |
| 20 | solution | 120 | 1.9 | 0.2 | 0.05 | neg | 350 | 9.1 |
| 22 | solution | 180 | 1.5 | 0.2 | 0.05 | neg | 350 | 9.0 |

Test results demonstrate that the amount of iron goes down in the solution. Furthermore, no hemoglobin was found after 30 seconds of microplasma treatment. Protein traces were identified by biuret method and Lowry method, but they appear to be the destroyed protein remnants in the solution, while blood protein is destroyed, as far as hemoglobin is negative.

Solution samples and catheter samples (No. 30-50) were examined at the microbiological laboratory of Tomsk Medical University. Test results are given in table 7.

**Table 7.**

| Sample No. | | Time of microplasma treatment, s | Protein concentration (micro mole/ml) | Hemoglobin content, micromole/l (overstated) | Intensity of Soret band compared to max (sample 30), % | Comments |
|---|---|---|---|---|---|---|
| 30 | solution | | 79.7 | 6.47 | 100 | Hemoglobin content is overstated. |
| 31 | solution | 30 | 72.0 | 6.31 | 97 | |
| 32 | solution | 60 | 85.8 | 5.71 | 88 | |
| 33 | solution | 90 | 105.9 | 6.31 | 98 | |
| 34 | solution | 120 | 103.7 | 6.50 | 100 | |
| 35 | solution | 150 | 93.7 | 6.19 | 96 | |
| 36 | solution | 180 | 82.6 | 5.45 | 84 | |
| 37 | solution | 240 | 75.3 | 5.12 | 79 | |
| 38 | solution | 300 | 70.8 | 5.08 | 78 | |
| 39 | catheter | 300 | - | - | | Below analysis accuracy threshold. |
| 40 | clear solution | | 4.2 | - | | No hemoglobin absorption bands in the spectrum. Low content of admixtures. |
| 41 | solution | | 49.6 | - | | Practically no hemoglobin absorption bands are revealed in the spectrum. |
| 42 | solution | 30 | 49.7 | - | | |
| 43 | solution | 60 | 57.6 | - | | |
| 44 | solution | 90 | 49.6 | - | | |
| 45 | solution | 120 | 58.6 | - | | |
| 46 | catheter | 120 | - | - | | Below analysis accuracy threshold. |
| 47 | solution | 180 | 57.8 | - | | Practically no hemoglobin absorption bands are revealed in the spectrum. |
| 48 | solution | 240 | 63.3 | - | | |
| 49 | solution | 300 | 69.1 | - | | |
| 50 | catheter | 300 | - | - | | Below analysis accuracy threshold. |

Analysis results for samples 31-38 show that the range contains characteristic hemoglobin absorption band belonging to the porphyrin part of its molecule (Soret band), as well as characteristic bands in the range of 600-500 nm. As far as analysis was carried out more than 72 hours after taking the samples, quantitative value (micromole/l of hemoglobin) is likely to be overstated; hence it makes more sense to pay attention to intensity ratio (%).

### Conclusions:

In order to analysis instrument contaminants, the samples of catheter (No. 39, 46, 50) were put into a microtube and 2 ml of water were added; they were treated in the ultrasonic unit for 2 minutes at 22 kHz, then the liquid from catheter was transferred into the bulk volume with a micropipette.

No traces of protein and hemoglobin were found on the surface of catheter samples (No. 39, 46, 50) after treating in the ultrasonic unit at 22 kHz for 2 minutes. As far as initially the samples were contaminated with dry blood, this proves that cleaning and disinfection were efficient.

In solutions of the samples No. 40-50 protein concentrations ranges from 49.10⁻⁶ g/ml to 69.10⁻⁶ g/ml. In the sample solutions No. 40-50 no traces of hemoglobin were found, hence protein present in the solution of these samples is actually represented by secondary and tertiary forms of protein, produced after treatment and protein destruction.

The testing of solutions and samples allows talking about reliable cleaning and disinfection of instruments.

### Example 4. Testing for compliance with the European standards

In accordance with the European standards, in order to identify the traces of protein, one uses highly sensitive express technique for protein trace identification recommended by EN ISO 15883 and British guidelines UK HTM2030 - Clean-Trace™Protein Test produced by 3M™ and designed to reveal the traces of protein on the studied surface. The test is used to assess the degree of protein contamination of a medical instrument after its treatment in the washing machine / disinfection unit. The test is to be applied after cleaning, in order to assess whether the instrument has been sufficiently cleaned for efficient sterilization.

One used the device for cleaning, disinfection and sterilization (Fig. 7) with one power supply PS1 and one additional vertical chamber MP2 (Fig. 3) to treat Nelaton urinary catheters contaminated with blood inside and outside and dried for an hour.

Treatment mode: pulse voltage of 480 V, anode-cathode mode with anode current density of 300 A/dm² and cathode current density of 300 A/dm².

Microplasma treatment of the samples was carried out for a certain period of time (15, 30, 60, 120 s).

After each treatment period, catheter was taken out of electrolyte, 15 mm piece of catheter was cut off with clean scissors and placed into the case with Clean-Trace™Protein Test, adding 2 ml of sterile water from Sigma-Aldrich designed for research, in order to exclude or minimize alkaline effect of electrolyte (electrically conductive medium). The tested samples were heated in an incubator at 55⁰C for 15 minutes in accordance with the guidelines. Control was done using clear electrolyte.

Results interpretation. Coloring that emerges in the course of testing demonstrates the degree of contamination of the examined surface with traces of protein. Purity level of the studied surface can be evaluated comparing the coloring to the reference on the label of Clean-Trace™Protein Test.
- Green color indicates acceptable result - there are no contaminants, there is no need for additional measures
- Grey or purple indicates unsatisfactory result - there is a need to repeat the cleaning and retest

The results obtained are given in tables 8 and 9 and in Fig. 10 (a and b) and demonstrate that in the course of at least two minutes catheter sample surface gets completely cleared of protein traces.

**Table 8. Purity test results for catheter samples after microplasma treatment**

| Sample number | Time of microplasma treatment | Result of protein trace test | Sample purity |
|---|---|---|---|
| 1 | 15 | xxx - purple | dirty |
| 2 | 30 | xx - purple | dirty |
| 3 | 60 | xx - purple | dirty |
| 4 | 120 | v - green | pure |
| 5 | 180 | v - green | pure |

**Table 9. Purity test results of catheter sample after microplasma treatment**

| Sample number | Time of microplasma treatment | Result of protein trace test | Sample purity |
|---|---|---|---|
| 1 | 30 | xxx - purple | dirty |
| 2 | 60 | xx - light purple | dirty |
| 3 | 90 | v - green | pure |
| 4 | 120 | v - green | pure |
| 5 | 150 | v - green | pure |

Table 10 lists control purity tests of electrically conductive medium and contaminated catheter without microplasma treatment.

**Table 10. Control purity tests of electrically conductive medium (1-2) and contaminated catheter (3-4) without microplasma treatment.**

| Control sample | Time of microplasma treatment | Result of protein trace test | Sample purity |
|---|---|---|---|
| 1. 15 mm of clean sterile catheter without sterile water | 0 | v - green | pure |
| 2. 15 mm of cleaned catheter washed over by 2 ml of sterile water | 120 s | v - green | pure |
| 3. 15 mm of dirty catheter washed over by 2 ml of sterile water | 0 | xxx - purple | dirty |
| 4. Contaminated catheter was put into electrically conductive medium solution without microplasma treatment for 2 minutes, then 15 mm of this catheter were washed over by 2 ml of sterile water. | 0 | xxx - purple | dirty |

Based on the comprehensive study conclusion was made that the claimed device for cleaning, disinfection and sterilization ensures reliable cleaning and disinfection of medical instruments.

### Example 5. Cleaning, disinfection and sterilization of metallic and non-metallic surgical instruments at the Synergy Health Hospital, London, UK, using the claimed method of microplasma treatment.

In order to clean, disinfect and sterilize metallic and non-metallic instruments brought to the hospital after having been used in patient treatment, one used the main vertical chamber (Fig. 3) connected to one power supply PS1 and one additional microplasma chamber MP2 (Fig. 7). Treatment modes were analogous to the ones in example 4.

Microplasma treatment was carried out in the course of different time periods from 20 seconds to 2 minutes, whereas activated (in MP2) current-conductive medium was circulated along the instrument surface and inside each channel (Fig. 7). This was done for the following instruments: plastic urinary catheter, dental drill with carbide coating, diathermic dissector, pediatric cystoscope, surgical drain instrument, electric scalpel, clamp and other instruments, as well as dental air-turbine handpiece with four channels.

Table 11 and Fig. 10 list the results of analysis carried out using Clean-Trace™Protein Test. It was shown that 2 minutes of microplasma treatment are enough to remove traces of protein from outer and inner surfaces of the treated instruments.

**Table 11. Results of Clean-Trace™Protein Test analysis of Synergy Health Hospital medical instruments subjected to microplasma treatment**

| | Instrument name | Time of microplasma treatment, s | Clean-Trace™Protein Test | Instrument purity |
|---|---|---|---|---|
| 1. | Plastic urinary catheter | 45 | v - green | Clean |
| 2. | Dental drill with carbide coating | 30 | v - green | Clean |
| 3. | Diathermic dissector | 40 | v - green | Clean |
| 4. | Pediatric cystoscope | 30 | v - green | Clean |
| 5. | Surgical drain instrument | 40 | v - green | Clean |
| 6. | Scalpel | 120 | v - green | Clean |
| 7. | Clamp | 120 | v - green | |
| 8. | Surface and 4 channels of dental air-turbine handpiece | 120 | v - green | Clean |
| | Channel 1 | | v - green | Clean |
| | Channel 2 | | v - green | Clean |
| | Channel 3 | | v - green | Clean |
| | Channel 4 | | v - green | Clean |

It was shown that this method does not destroy the surface of instruments and channels and cleans the surface with time ranging from 30 to 120 seconds.

### Example 6. Application of step voltage to clean surgical instruments (metallic and non-metallic) with channels.

In order to clean extremely contaminated channels of metallic medical instruments, one used the main vertical microplasma chamber (Fig 3), where microplasma treatment was carried out in electrically conductive medium in the course of different time periods from 20 seconds to 2 minutes, whereas activated current-conducting medium was circulated along the instrument surface, and activated current-conducting medium was circulated inside each channel (Fig. 7) with additional activation in additional chamber for each channel. In addition, step voltage was applied to inlet and outlet of each channel. Step voltage of 1000 V from power supply PS1 ensures faster cleaning of inner channel surfaces. Operation algorithm in this case is analogous to the algorithm given in Fig. 8.

Step voltage accelerates the cleaning and disinfection process. When bacteria get to the area with step voltage, voltage difference is formed on the boundary of bacteria and it is equal to bacterium length multiplied by the value of step voltage. If voltage value formed by step voltage exceeds the value of membrane potential in bacterial cells, cell membranes are destroyed, which leads to the death of bacteria and viruses.

Table 12 lists the results of microplasma treatment of metallic and non-metallic medical instruments with channels with additional effect of step voltage accelerating the treatment. It was demonstrated that 10 seconds of microplasma treatment and effect of step voltage are not enough for efficient cleaning, disinfection and sterilization, but after 30 seconds and even more so after 50 seconds of such treatment the surfaces of the instrument and channels are clean, which is evidenced by the test results.

**Table 12. Results of cleaning and disinfection of the instrument with channels in case of additional effect of step voltage.**

| | Instrument name | Time of microplasma treatmen t, s | Solution color in Clean-Trace™Protein Test | Instrument purity |
|---|---|---|---|---|
| 1 | Urinary catheter, metal, male | 10 | xx - purple | dirty |
| | | 30 | v - green | clean |
| | | 50 | v - green | clean |
| 2 | Urinary catheter, metal, female | 10 | xx - purple | dirty |
| | | 30 | v - green | clean |
| | | 50 | v - green | clean |
| 3 | Urinary catheter, metal, for children | 10 | xx - purple | dirty |
| | | 30 | v - green | clean |
| | | 50 | v - green | clean |
| 4 | Urinary catheter, non-metal, male | 10 | xx - purple | dirty |
| | | 30 | v - green | clean |
| | | 50 | v - green | clean |
| 5 | Urinary catheter, non-metal, female | 10 | xx - purple | dirty |
| | | 30 | v - green | clean |
| | | 50 | v - green | clean |
| 6 | Urinary catheter, non-metal, for children | 10 | xx - purple | dirty |
| | | 30 | v - green | clean |
| | | 50 | v - green | clean |
| | Surface and 4 channels of dental air-turbine handpiece | 50 | v - green | clean |
| | Channel 1 | | v - green | clean |
| | Channel 2 | | v - green | clean |
| | Channel 3 | | v - green | clean |
| | Channel 4 | | v - green | clean |

### Example 7. Cleaning, disinfecting and sterilizing of porous medical articles (implants with porous coating).

Titanium plate with bioactive and hydroxyapatite porous coating (dimensions 15× 120× 2 mm) was subjected to cleaning and sterilization in the unit with one power source (Fig. 4) that has an additional vacuum pump (not shown in the figure) and leak-proof horizontal microplasma chamber (Fig. 1) with electrically conductive medium - water borate-phosphate solution (Table 1).

Operation mode of the power supply: microplasma discharges are generated on the electrodes at the voltage of 440 V, using pulse anode-cathode mode with current density of 250 A/dm².

After primary microplasma treatment in the course of 30 seconds, vacuum pump was switched on and pressure was reduced to partial pressure of electrically conductive medium vapors for 10 seconds. The treatment was repeated 3 times. Clean-Trace™Protein Test showed the absence of protein traces in pores, i.e. pulsing change of pressure from atmospheric to reduced pressure equal to partial pressure of electrically conductive medium water solvent vapors allows removing contaminants both from the implant surface and from the air inside the implant pores, while implant surface is not damaged, which can be observed visually and under microscope with 10-fold and 60-fold magnification.

Sterility tests were carried out earlier after microplasma treatment and disinfection of contaminated dental instruments (drills, burs, dental broaches, root canal fillers, root needles) taken from the dentist's table after patient treatment at the Tomsk Regional Dental Clinic, Tomsk Regional Center of the Russian State Committee for Sanitary and Epidemiological Oversight and Tomsk research and production association Virion (Research Institute of Vaccines and Sera).

Testing of dental drills for sterility and presence of occult blood at the Tomsk Regional Center of Russian State Committee for Sanitary and Epidemiological Oversight showed the absence of mesophilic aerobes and facultative anaerobes.

Instruments were also examined at the Tomsk research and production association Virion (Research Institute of Vaccines and Sera) and tested for pyrogenicity, sterility and toxicity. Sterility control was performed relying on methodology guidelines 42-1844-88 (temporary pharmacopoeia chapter) - "Sterility Testing" in nutritional media: thioglycol, sabouraud (broth), beef extract agar (solid), Sabouraud (solid), Tarozzi, meat-peptone broth, and sugar broth. Culture was inoculated 48 hours after the microplasma treatment. It was shown that microplasma treatment method ensures sterility of dental instruments.

Furthermore, pyrogenicity tests were run relying on the "Methodology for pyrogenicity control of sterile disposable polymeric syringes" developed by the Central Research Institute of Hematology and Blood Transfusion. Test subjects were three rabbits with 1600, 1650 and 1700 grams of body weight. The dose of electrolyte solution was 10 ml per one kg. Temperature control was performed every hour in the course of three hours after solution injection. Control showed apyrogenicity of electrically conductive medium solutions after microplasma treatment of the medical instrument.

Toxicity studies were carried out in accordance with the methodology for toxicity control of polymeric disposable injection syringes developed by the Russian Research Institute of Medical Equipment. Control was carried out using a control and an experimental groups (8 subjects in each) of linear mice (white line) with body weight of 18-21 grams. Their overall condition was under control and changes in animals' liver, spleen and kidney (weight/g) were evaluated. Electrolyte solutions in which drills were treated with microplasma method have no toxic effect on the overall condition and above-mentioned organs of the animals.

Sterility testing of medical metallic and non-metallic instruments and electrically conductive medium solutions. 48 hours after microplasma treatment the culture was inoculated into nutritional media: thioglycol, Sabouraud (broth), beef extract agar (solid), Sabouraud (solid), Tarozzi, meat-peptone broth, and sugar broth. As far as nutritional media: Sabouraud (broth), beef extract agar (solid), Sabouraud (solid), meet-peptone broth, and sugar broth had a destructive effect on metallic instruments, they were used for non-metallic ones, and for metallic instruments sterility testing was carried out in thioglycol and Tarozzi media that do not destroy metallic instruments and do not require reinoculation. Sterility testing showed that all metallic and non-metallic instruments that underwent microplasma treatment from 50 to 120 seconds are sterile.

### INDUSTRIAL APPLICABILITY

Sterility testing showed that the claimed microplasma treatment method allows for efficient cleaning, disinfecting and sterilizing of medical articles and instruments and at the same time is applicable for treatment of both metallic and non-metallic articles and instruments, including non-metallic instruments with metallic parts and instruments with channels and cavities, as well as articles and instruments with porous surfaces, which allows for its use in surgery, specialized hospital departments, and dental practice; it can also be used in beauty and hairdressing salons etc.

## Claims

1. A method for cleaning, disinfection and sterilization of medical and hygiene articles or instruments, in which said articles or instruments are put into a main microplasma chamber (1) filled with electrically conductive medium and are subjected to an indirect effect of electric current, which is fed to electrodes (2, 2') located in the main microplasma chamber (1) and connected to a power supply (4), wherein said articles or instruments are placed in the immediate proximity of the electrodes: at least one anode (2) and cathode (2') and the indirect effect of electric current is provided under condition of generation of microplasma discharges on all electrodes (2, 2') simultaneously, **characterized in that** the process of cleaning disinfection and sterilization in the main microplasma chamber (1) is intensified by circulation of the electrically conductive medium to an additional microplasma chamber (5) for preparing an additionally activated electrically conductive medium, wherein electrodes (9, 9') are placed in the additional microplasma chamber (5) to generate microplasma discharges.

2. The method of claim 1, **characterized in that** for cleaning, disinfection and sterilization of said articles or instruments with channels, primarily endoscopes and dental air-turbine handpieces, electrically conductive medium is additionally circulated in the main microplasma chamber (1) and/or in the channels of said articles or instruments.

3. The method of claim 1, **characterized in that** for cleaning, disinfecting and sterilizing of said articles or instruments with porous surface, primarily implants, the indirect effect of electric current is provided under condition of reduced pressure equal to partial pressure of electrically conductive medium vapors.

4. The method of claim 1 or 3, **characterized in that** said articles or instruments are additionally subjected to an effect of step voltage - voltage drop between two additional electrodes (8, 8'), one of which is placed in the main microplasma chamber (1) at an inlet of activated electrically conductive medium, while the other is placed at an outlet of electrically conductive medium from the main microplasma chamber (1).

5. The method of claim 2, **characterized in that** said articles or instruments with channels are additionally subjected to an effect of step voltage - voltage drop created by two additional electrodes (8, 8'), one of which is placed at an inlet of activated electrically conductive medium into an instruments channel or the main microplasma chamber (1) and the other is placed at an outlet of electrically conductive medium from the channel or the main microplasma chamber (1).

6. The method of claim 1 or 3, **characterized in that** metallic medical or hygiene articles or instruments are additionally subjected to a direct effect of electric current by connecting them to one of the electrodes - anode (2) or cathode (2').

7. The method of claim 1 or 2 or 3, **characterized in that** microplasma discharges are generated on the electrodes represented by multiple alternating anodes (2) and cathodes (2') in a form of metallic plates of the same size made of aluminum, titanium, zircounium or their alloys, that are uniformly distributed throughout the main microplasma chamber (1) volume and the electrodes (2, 2') are insulated from each other with dielectric material and form a single electrode array.

8. The method of claim 1 or 2 or 3, **characterized in that** said articles or instruments are put into the main microplasma chamber (1) on guides (3) above or between the electrodes: anode (2) and cathode (2'), or in one of the electrodes (2, 2') or in the volume of the electrode array.

9. The method of claim 1, **characterized in that** in order to ensure cyclic impact of electrically conductive medium upon said articles or instruments, its pulsing feed is provided.

10. A device for cleaning, disinfection and sterilization of medical and hygiene articles or instruments containing at least one main microplasma chamber (1) containing at least two electrodes: anode (2) and cathode (2') connected to a pulse power supply (4) and designed for placement of said articles or instruments and electrically conductive medium, **characterized in that** the device further comprises a circuit of the activated conductive medium comprising an additional microplasma chamber (5) connected to the main microplasma chamber (1) for preparing an additionally activated electrically conductive medium, wherein electrodes (9, 9') are placed in the additional microplasma chamber (5) to generated microplasma discharges.

11. The device of claim 10, **characterized in that** for cleaning, disinfection and sterilization of articles or instruments with elongated channels, it has at least one separate additional microplasma chamber for each channel, designed for additional activation of electrically conductive medium, wherein electrodes (9, 9') are placed to generate microplasma discharges on them and wherein the system of circulation contains at least one filter (6) for mechanical cleaning of electrically conductive medium located at the outlet of the main microplasma chamber (1) and medical instruments channels and/or to the additional microplasma chamber (5), as well as a system of pumps (11) and valves (10) to feed the cleaned electrically conductive medium into said additional microplasma chamber (5).

12. The device of claim 10 or claim 11, **characterized in that** in order to create step voltage - voltage drop in the main microplasma chamber (1) to intensify the cleaning, disinfection and sterilization process, additional electrodes (8, 8') are placed either at an inlet and outlet of the main microplasma chamber (1) or at an inlet and outlet of each channel in case of treatment of articles or instruments with channels, in order to intensify the cleaning, disinfection and sterilization of each channel of said articles or instruments.

13. The device of claim 10, **characterized in that** the main microplasma chamber (1) has a housing, where multiple electrodes (2, 2') of the same size are located as alternating anodes and cathodes, shaped as plates and insulated with dielectric material, forming a single array with those electrodes (2, 2').

14. The device of claim 13, **characterized in that** there are guides (3) in the electrode array designed to place said articles or instruments located over the array in the immediate proximity of the electrodes.

15. The device of any of the claims 10-14, **characterized in that** the electrodes (2, 2', 9, 9') with which the main and additional microplasma chambers (1,5) are equipped are made of valve metals, such as aluminum, titanium, zirconium or their alloys with oxide coating on their surface or without a coating.

## Patentansprüche

1. Verfahren zur Reinigung, Desinfektion und Sterilisation von medizinischen und Hygieneartikeln oder -instrumenten, bei dem die Artikel oder Instrumente in eine Haupt-Mikroplasmakammer (1) eingeführt werden, die mit einem elektrisch leitfähigen Medium gefüllt ist, und einer indirekten Wirkung des elektrischen Stroms ausgesetzt werden, der Elektroden (2, 2') zugeführt wird, die in der Haupt-Mikroplasmakammer (1) angeordnet und an eine Stromquelle (4) angeschlossen sind, wobei die Artikel oder Instrumente in unmittelbarer Nähe der Elektroden angeordnet werden: Wenigstens eine Anode (2) und Kathode (2'), und die indirekte Wirkung des elektrischen Stroms wird in einem Zustand erzeugt, bei dem Mikroplasma-Entladungen an allen Elektroden (2, 2') gleichzeitig erzeugt werden, **dadurch gekennzeichnet, dass** der Prozess der Reinigung, Desinfektion und Sterilisation in der Haupt-Mikroplasmakammer (1) durch eine Zirkulation des elektrisch leitfähigen Mediums zu einer zusätzlichen Mikroplasmakammer (5) intensiviert wird, um ein zusätzlich aktiviertes elektrisch leitfähiges Medium zu herzustellen, wobei Elektroden (9, 9') in der zusätzlichen Mikroplasmakammer (5) angeordnet werden, um Mikroplasma-Entladungen zu erzeugen.

2. Verfahren nach Anspruch 1, **dadurch gekennzeichnet, dass** zur Reinigung, Desinfektion und Sterilisation der Artikel oder Instrumente mit Kanälen, hauptsächlich von Endoskopen und Handstücken für zahnärztliche Luftturbinen, elektrisch leitfähiges Medium zusätzlich in der Haupt-Mikroplasmakammer (1) und/oder in den Kanälen der Artikel oder Instrumente zirkuliert wird.

3. Verfahren nach Anspruch 1, **dadurch gekennzeichnet, dass** zur Reiniging, Desinfektion und Sterilisation der Artikel oder Instrumente mit poröser Oberfläche, hauptsächlich von Implantaten, die indirekte Wirkung des elektrischen Stroms in einem Zustand eines reduzierten Drucks erzeugt wird, der gleich dem Partialdruck der Dämpfe des elektrisch leitfähigen Mediums ist.

4. Verfahren nach einem der Ansprüche 1 oder 3, **dadurch gekennzeichnet, dass** die Artikel oder Instrumente zusätzlich einer Wirkung einer Schrittspannung unterzogen werden, d.h. eines Spannungsabfalls zwischen zwei zusätzlichen Elektroden (8, 8'), von denen eine in der Haupt-Mikroplasmakammer (1) an einem Einlass des aktivierten elektrisch leitfähigen Mediums angeordnet ist, während die andere an einem Auslass des elektrisch leitfähigen Mediums aus der Haupt-Mikroplasmakammer (1) angeordnet ist.

5. Verfahren nach Anspruch 2, **dadurch gekennzeichnet, dass** die Artikel oder Instrumente mit Kanälen zusätzlich einer Wirkung einer Schrittspannung unterzogen werden, d.h. eines Spannungsabfalls, der durch zwei zusätzliche Elektroden (8, 8') verursacht wird, von denen eine an einem Einlass des aktivierten elektrisch leitfähigen Mediums in einen Instrumentenkanal oder die Haupt-Mikroplasmakammer (1) und die andere an einem Auslass des elektrisch leitfähigen Mediums aus dem Kanal oder der Haupt-Mikroplasmakammer (1) angeordnet ist.

6. Verfahren nach Anspruch 1 oder 3, **dadurch gekennzeichnet, dass** metallische medizinische oder Hygieneartikel oder -instrumente zusätzlich einer direkten Wirkung des elektrischen Stroms unterzogen werden, indem sie mit einer der Elektroden, d.h. der Anode (2) oder der Kathode (2') verbunden werden.

7. Verfahren nach Anspruch 1, 2 oder 3, **dadurch gekennzeichnet, dass** Mikroplasma-Entladungen an den Elektroden erzeugt werden, die durch eine Vielzahl von alternierenden Anoden (2) und Kathoden (2') in Form von Metallplatten gleicher Größe repräsentiert sind, die aus Aluminium, Titan, Zirkonium oder deren Legierungen hergestellt und gleichmäßig in dem Hohlraum der Haupt-Mikroplasmakammer (1) verteilt sind, und die Elektroden (2, 2') mit dielektrischem Material voneinander isoliert sind und eine Einzelelektrodenanordnung bilden.

8. Verfahren nach Anspruch 1, 2 oder 3, **dadurch gekennzeichnet, dass** die Artikel oder Instrumente auf Führungen (3) über oder zwischen den Elektroden: der Anode (2) und der Kathode (2'), oder in einer der Elektroden (2, 2') oder in dem Hohlraum der Elektrodenanordnung in die Haupt-Mikroplasmakammer (1) eingeführt werden.

9. Verfahren nach Anspruch 1, **dadurch gekennzeichnet, dass**, um eine zyklische Einwirkung des elektrisch leitfähigen Mediums auf die Artikel oder Instrumente zu gewährleisten, dieses pulsweise zugeführt wird.

10. Vorrichtung zur Reinigung, Desinfektion und Sterilisation von medizinischen und Hygieneartikeln oder -instrumenten, die wenigstens eine Haupt-Mikroplasmakammer (1) aufweist, die wenigstens zwei Elektroden aufweist: eine Anode (2) und eine Kathode (2'), die mit einer Pulsstromversorgung (4) verbunden sind, und die für eine Anordnung der Artikel oder Instrumente und eines elektrisch leitfähigen Mediums ausgelegt ist, **dadurch gekennzeichnet, dass** die Vorrichtung weiterhin einen Kreislauf des aktivierten leitfähigen Mediums umfasst, der eine zusätzliche Mikroplasmakammer (5) umfasst, die mit der Haupt-Mikroplasmakammer (1) verbunden ist, um ein zusätzlich aktiviertes elektrisch leitfähiges Medium herzustellen, wobei Elektroden (9, 9') in der zusätzlichen Mikroplasmakammer (5) angeordnet sind, um Mikroplasma-Entladungen zu erzeugen.

11. Vorrichtung nach Anspruch 10, **dadurch gekennzeichnet, dass** sie zur Reinigung, Desinfektion und Sterilisation von Artikeln oder Instrumenten mit langgestreckten Kanälen wenigstens eine separate zusätzliche Mikroplasmakammer für jeden Kanal aufweist, die für eine zusätzliche Aktivierung des elektrisch leitfähigen Mediums ausgelegt ist, wobei die Elektroden (9, 9') angeordnet sind, um Mikroplasma-Entladungen darauf zu erzeugen, und wobei das Zirkulationssystem wenigstens einen Filter (6) für eine mechanische Reinigung des elektrisch leitfähigen Mediums, der an dem Auslass der Haupt-Mikroplasmakammer (1) und den Kanälen der medizinischen Instrumente und/oder der zusätzlichen Mikroplasmakammer (5) angeordnet ist, sowie ein System von Pumpen (11) und Ventilen (10) aufweist, um das gereinigte elektrisch leitfähige Medium in die zusätzliche Mikroplasmakammer (5) zu leiten.

12. Vorrichtung nach Anspruch 10 oder Anspruch 11, **dadurch gekennzeichnet, dass** zur Erzeugung einer Schrittspannung, d.h. eines Spannungsabfalls in der Haupt-Mikroplasmakammer (1), zur Intensivierung des Reinigungs-, Desinfektions- und Sterilisationsprozesses zusätzliche Elektroden (8, 8') entweder an einem Einlass und einem Auslass der Haupt-Mikroplasmakammer (1) oder an einem Einlass und einem Auslass jedes Kanals angeordnet sind, falls Artikel oder Instrumente mit Kanälen zu behandeln sind, um die Reinigung, Desinfektion und Sterilisation jedes Kanals der Artikel oder Instrumente zu intensivieren.

13. Vorrichtung nach Anspruch 10, **dadurch gekennzeichnet, dass** die Haupt-Mikroplasmakammer (1) ein Gehäuse aufweist, wo eine Vielzahl von Elektroden (2, 2') von gleicher Größe als alternierende Anoden und Kathoden angeordnet sind, die plattenförmig und mit dielektrischem Material isoliert sind, wobei eine einzelne Anordnung mit diesen Elektroden (2, 2') gebildet wird.

14. Vorrichtung nach Anspruch 13, **dadurch gekennzeichnet, dass** Führungen (3) in der Elektrodenanordnung angeordnet sind, die dafür ausgelegt sind, die Artikel oder Instrumente, die über der Anordnung angeordnet sind, in unmittelbarer Nähe der Elektroden anzuordnen.

15. Vorrichtung nach einem der Ansprüche 10 bis 14, **dadurch gekennzeichnet, dass** die Elektroden (2, 2', 9, 9'), mit denen die Haupt-Mikroplasmakammer und die zusätzliche Mikroplasmakammer (1, 5) ausgestattet sind, aus Ventilmetallen wie Aluminium, Titan, Zirkonium oder deren Legierungen mit einer Oxidbeschichtung an ihrer Oberfläche oder ohne eine Beschichtung hergestellt sind.

## Revendications

1. Procédé pour le nettoyage, la désinfection et la stérilisation d'articles ou d'instruments médicaux et d'hygiène, dans lequel lesdits articles ou instruments sont placés dans une chambre de microplasma principale (1) remplie avec un milieu électriquement conducteur et sont soumis à un effet indirect de courant électrique, qui est alimenté aux électrodes (2, 2') situées dans la chambre de microplasma principale (1) et connectées à une alimentation (4), où lesdits articles ou instruments sont placés à proximité immédiate des électrodes : au moins une anode (2) et une cathode (2') et l'effet indirect de courant électrique est fourni dans une condition de production de décharges de microplasma sur toutes les électrodes (2, 2') simultanément, **caractérisé en ce que** le processus de nettoyage, de désinfection et de stérilisation dans la chambre de microplasma principale (1) est intensifié par la circulation du milieu électriquement conducteur à une chambre de microplasma (5) supplémentaire pour préparer un milieu électriquement conducteur additionnellement activé, où des électrodes (9, 9') sont placées dans la chambre de microplasma (5) supplémentaire pour produire des décharges de microplasma.

2. Procédé selon la revendication 1, **caractérisé en ce que** pour le nettoyage, la désinfection et la stérilisation desdits articles ou instruments avec des canaux, principalement des endoscopes et des pièces à main à turbine à air dentaires, le milieu électriquement conducteur est de plus mis en circulation dans la chambre de microplasma principale (1) et/ou dans les canaux desdits articles ou instruments.

3. Procédé selon la revendication 1, **caractérisé en ce que** pour le nettoyage, la désinfection et la stérilisation desdits articles ou instruments avec une surface poreuse, principalement des implants, l'effet indirect du courant électrique est fourni dans une condition de pression réduite égale à la pression partielle des vapeurs du milieu électriquement conducteur.

4. Procédé selon la revendication 1 ou 3, **caractérisé en ce que** lesdits articles ou instruments sont soumis de plus à une tension par étape - chute de tension entre deux électrodes supplémentaires (8, 8'), dont l'une est placée dans la chambre de microplasma principale (1) à une entrée du milieu électriquement conducteur activé, alors que l'autre est placée à une sortie du milieu électriquement conducteur de la chambre de microplasma principale (1).

5. Procédé selon la revendication 2, **caractérisé en ce que** lesdits articles ou instruments avec des canaux sont de plus soumis à un effet de tension par étape - chute de tension créé par deux électrodes supplémentaires (8, 8'), dont l'une est placée à une entrée du milieu électriquement conducteur activé dans un canal d'instruments ou de la chambre de microplasma principale (1) et l'autre est placée à une sortie du milieu électriquement conducteur du canal ou de la chambre de microplasma principale (1).

6. Procédé selon la revendication 1 ou 3, **caractérisé en ce que** les articles ou instruments médicaux ou d'hygiène métalliques sont de plus soumis à un effet direct du courant électrique en les connectant à une des électrodes - anode (2) ou cathode (2').

7. Procédé selon la revendication 1 ou 2 ou 3, **caractérisé en ce que** les décharges de microplasma sont produites sur les électrodes représentées par des anodes (2) et des cathodes (2') en alternance multiples sous forme de plaques métalliques de la même taille constituées d'aluminium, de titane, de zirconium ou de leurs alliages, qui sont uniformément distribuées dans l'ensemble du volume de la chambre de microplasma principale (1) et les électrodes (2, 2') sont isolées l'une de l'autre avec un matériau diélectrique et forment un simple réseau d'électrodes.

8. Procédé selon la revendication 1, 2 ou 3, **caractérisé en ce que** les articles ou instruments sont placés dans la chambre de microplasma principale (1) sur des guides (3) au-dessus ou entre les électrodes : l'anode (2) et la cathode (2'), ou dans l'une des électrodes (2, 2') ou dans le volume du réseau d'électrodes.

9. Procédé selon la revendication 1, **caractérisé en ce que** pour assurer un impact cyclique du milieu électriquement conducteur sur lesdits articles ou instruments, son alimentation d'impulsion est fournie.

10. Dispositif pour le nettoyage, la désinfection et la stérilisation d'articles ou d'instruments médicaux et d'hygiène contenant au moins une chambre de microplasma principale (1) contenant au moins deux électrodes : une anode (2) et une cathode (2') connectées à une alimentation à impulsion (4) et conçues pour le placement desdits articles ou instruments et de milieu électriquement conducteur, **caractérisé en ce que** le dispositif comprend en outre un circuit du milieu conducteur activé comprenant une chambre de microplasma (5) supplémentaire connectée à la chambre de microplasma principale (1) pour préparer un milieu électriquement conducteur activé supplémentaire, où des électrodes (9, 9') sont placées dans la chambre de microplasma (5) supplémentaire pour produire des décharges de microplasma.

11. Dispositif selon la revendication 10, **caractérisé en ce que** pour le nettoyage, la désinfection et la stérilisation d'articles ou d'instruments avec des canaux allongés, il a au moins une chambre de microplasma supplémentaire séparée pour chaque canal, conçue pour une activation supplémentaire du milieu électriquement conducteur, où des électrodes (9, 9') sont placées pour produire des décharges de microplasma dessus et où le système de circulation contient au moins un filtre (6) pour le nettoyage mécanique du milieu électriquement conducteur situé à la sortie de la chambre de microplasma principale (1) et les canaux d'instruments médicaux et/ou la chambre de microplasma (5) supplémentaire, ainsi qu'un système de pompes (11) et de valves (10) pour alimenter le milieu électriquement conducteur nettoyé dans ladite chambre de microplasma (5) supplémentaire.

12. Dispositif selon la revendication 10 ou la revendication 11, **caractérisé en ce que** pour créer une tension par étape - chute de tension dans la chambre de microplasma principale (1) pour intensifier le processus de nettoyage, de désinfection et de stérilisation, des électrodes supplémentaires (8, 8') sont placées à une entrée et une sortie de la chambre de microplasma principale (1) ou à une entrée et une sortie de chaque canal dans le cas de traitement d'articles ou d'instruments avec des canaux, pour intensifier le nettoyage, la désinfection et la stérilisation de chaque canal desdits articles ou instruments.

13. Dispositif selon la revendication 10, **caractérisé en ce que** la chambre de microplasma principale (1) a un logement, où de multiples électrodes (2, 2') de la même taille sont situées comme anodes et cathodes en alternance, formées en plaques et isolées avec un matériau diélectrique, formant un simple réseau avec ces électrodes (2, 2').

14. Dispositif selon la revendication 13, **caractérisé en ce qu'**il y a des guides dans le réseau d'électrodes conçus pour placer lesdits articles ou instruments situés sur le réseau à proximité immédiate des électrodes.

15. Dispositif selon l'une quelconque des revendications 10 à 14, **caractérisé en ce que** les électrodes (2, 2', 9, 9') avec lesquelles les chambres de microplasma principale et supplémentaire (1, 5) sont équipées sont constituées de métaux de valve, tels que l'aluminium, le titane, le zirconium ou leurs alliages avec un revêtement d'oxyde sur leur surface ou sans revêtement.
